# EUROPEAN PATENT APPLICATION

(11) **EP 4 736 760 A2**
(43) Date of publication of application: **06.05.2026**
(21) Application number: 26166124.3
(22) Date of filing: 22.02.2023
(51) Int. Cl.: A61B 5/145

(54) **SYSTEMS AND METHODS FOR MULTI-ANALYTE SENSING**

(30) Priority: 22.02.2022 US 202263268334 P
(62) Divisional of application: 23711391.5
(71) Applicant: DexCom, Inc., San Diego, CA 92121 (US)
(72) Inventor: CHENG, Kevin Ka Wing, San Diego, California, 92121 (US); HEADEN, Devon M., San Diego, California, 92121 (US); AN, Qi, San Diego, California, 92121 (US); DAMLE, Samir Sudhir, San Diego, California, 92121 (US); APOLLO, Nicholas Vincent, San Diego, California, 92121 (US); LIONG, Sylvie, San Diego, California, 92121 (US); VANRENTERGHEM, Hadley Faith, San Diego, California, 92121 (US); HELAYHEL, Mohamed R., San Diego, California, 92121 (US); SIMPSON, Peter Charles, San Diego, California, 92121 (US); FRANK, Spencer Troy, San Diego, California, 92121 (US)
(74) Representative: Crowell & Moring U.K. LLP

(57) **Abstract**

An apparatus includes an analyte sensor, a memory, and a processor. The processor monitors, using the analyte sensor, an analyte of a patient during a time period to obtain measured analyte data for the analyte and monitors other measured sensor data indicative of a physiological state of the patient during the time period. The processor also determines, based on the physiological state of the patient during the time period, expected analyte data for the analyte and determines a correction factor based on the expected analyte data and the measured analyte data. The correction factor is indicative of an error in calibration of the analyte sensor. The processor also determines whether recalibration of the analyte sensor is possible. If recalibration is possible, the processor recalibrates the analyte sensor based on the correction factor, and if recalibration is not possible, the processor recommends, to the patient, to replace the analyte sensor.

## Description

### CROSS-REFERENCE TO RELATED APPLICATIONS

This application claims priority to and benefit of U.S. Provisional Patent Application No. 63/268,334, filed February 22, 2022, which is hereby assigned to the assignee hereof and hereby expressly incorporated by reference in its entirety as if fully set forth below and for all applicable purposes.

### BACKGROUND

Diabetes mellitus is a metabolic condition relating to the production or use of insulin by the body. Insulin is a hormone that allows the body to use glucose for energy, or store glucose as fat.

When a person eats a meal that contains carbohydrates, the digestive system absorbs nutrients, ultimately depositing glucose in the person's blood. Blood glucose can be used for energy or stored as fat. The body normally maintains blood glucose levels in a range that provides sufficient energy to support bodily functions and avoids problems that can arise when glucose levels are too high, or too low. Regulation of blood glucose levels depends on the production and use of insulin, which regulates the movement of blood glucose into cells.

When the body does not produce enough insulin, or when the body is unable to effectively use insulin that is present, blood sugar levels can elevate beyond normal ranges. The state of having a higher than normal blood sugar level is called "hyperglycemia." Chronic hyperglycemia can lead to a number of health problems, such as cardiovascular disease, cataract and other eye problems, nerve damage (neuropathy), skin ulcers, and kidney damage. Hyperglycemia can also lead to acute problems, such as diabetic ketoacidosis-a state in which the body becomes excessively acidic due to the production of excess ketones, or body acids. The state of having lower than normal blood glucose levels is called "hypoglycemia." Severe hypoglycemia can lead to damage of the heart muscle, neurocognitive dysfunction, and in certain cases, acute crises that can result in seizures or even death.

A patient living with diabetes can receive insulin to manage blood glucose levels. Insulin can be received, for example, through a manual injection with a needle. Wearable insulin pumps are also available. Diet and exercise also affect blood glucose levels.

Diabetes conditions are sometimes referred to as "Type 1" and "Type 2". A Type 1 diabetes patient is typically able to use insulin when it is present, but the body is unable to produce sufficient amounts of insulin, because of a problem with the insulin-producing beta cells of the pancreas. A Type 2 diabetes patient may produce some insulin, but the patient has become "insulin resistant" due to a reduced sensitivity to insulin. The result is that even though insulin is present in the body, the insulin is not sufficiently used by the patient's body to effectively regulate blood sugar levels.

Patients with diabetes can benefit from real-time diabetes management guidance, as determined based on a physiological state of the patient, in order to stay within a target glucose range and avoid physical complications. In certain cases, the physiological state of the patient is determined using diagnostics systems that measure glucose levels, which inform the identification and/or prediction of adverse glycemic events, such as hyperglycemia and hypoglycemia, and the type of guidance provided to the patient.

For example, such diagnostics systems may utilize a continuous glucose monitor (CGM) to measure a patient's glucose levels over time. The measured glucose levels may then be processed by the diagnostics system to identify and/or predict adverse glycemic events, and/or to provide guidance to the patient for treatment and or actions to abate or prevent the occurrence of such adverse glycemic events. For example, trends, statistics, or other metrics may be derived from the glucose levels and used to identify and/or predict adverse glycemic events. Or, in certain cases, the glucose levels themselves may be used to identify and/or predict adverse glycemic events.

Management of diabetes, however, presents many challenges for patients, clinicians, and caregivers, as a confluence of various factors can impact a patient's glucose levels, thus affecting the accuracy of glycemic event prediction and the guidance provided by diagnostics systems. As such, in order to provide a more comprehensive or accurate characterization of the patient's physiological state at any given moment, a plurality of different analytes, including glucose, may be monitored. The monitoring of one or more of multiple analytes, in addition to or in alternative to glucose, may provide a more complete picture of the physiological state of the patient, since multiple analytes may affect or be affected by the same physiological events/conditions, and/or may affect or be affected by related physiological events/conditions. Examples of such analytes include, ketones, lactate, insulin, electrolytes, creatinine, as well as a number of other biomarkers including proteins, metabolites, and nucleic acids...

Yet, simultaneous monitoring of multiple analytes creates many new challenges in itself, as doing so involves highly-involved mechanisms for sensor calibration, signal transmission and differentiation, artifact detection, etc. For example, the utilization of multiple sensors for multi-analyte sensing may lead to cross-talk between individual sensors, thus negatively affecting sensing accuracy and/or calibration thereof. Such cross-talk may be a function of sensor membrane properties, enzyme activity/immobilization, electrode surface defects, etc. Further, utilization of systems with multiple sensors (e.g., multiple potentiostats) may present various hardware and/or power consumption (e.g., battery life) concerns that are typically associated with increasing hardware and/or software complexity.

Moreover, when sensing multiple analytes with a signal multi-analyte sensor, the sensor may consume more local oxygen as compared to a single-analyte sensor, and thus, such a multi-analyte sensor is more likely to be oxygen-limited rather than diffusion-limited. And, when using a common resistance layer for multiple analytes in a multi-analyte sensor, maintaining selectivity and diffusivity for each analyte is challenging. Another unique challenge for multi-analyte sensor arises from pairs of analytes that locally interact with each other. In such examples, the local interaction between the analytes may not accurately reflect systemic changes of either analyte, leading to inaccurate characterizations of sensed analyte levels and/or trends.

Accordingly, there is a need in the art for improved systems and methods for multi-analyte monitoring for improved disease management support.

### BRIEF DESCRIPTION OF THE DRAWINGS

So that the manner in which the above-recited features of the present disclosure can be understood in detail, a more particular description, briefly summarized above, may be had by reference to aspects, some of which are illustrated in the drawings. It is to be noted, however, that the appended drawings illustrate only certain typical aspects of this disclosure and are therefore not to be considered limiting of its scope, for the description may admit to other equally effective aspects.
**FIG. 1** illustrates aspects of an example disease management system used in connection with implementing embodiments of the present disclosure.
**FIG. 2** is a diagram conceptually illustrating an example continuous analyte monitoring system including example continuous analyte sensor(s) with sensor electronics, in accordance with certain aspects of the present disclosure.
**FIGs. 3A-3B** schematically illustrate example configurations of a continuous analyte sensor with sensor electronics for use by the disease management system of **FIG. 1****,** according to some embodiments disclosed herein.
**FIG. 3C** schematically illustrates an example sensing mechanism of the continuous analyte sensor of **FIG. 3B****,** according to some embodiments disclosed herein.
**FIG. 3D** graphically illustrates an example multiplex sensing method for use with the continuous analyte sensors of **FIGs. 3A-3B****,** according to some embodiments disclosed herein.
**FIGs. 4A-4B** illustrate flow diagrams depicting example methods for automatic correction of a factory-calibrated continuous analyte sensor, according to some embodiments disclosed herein.
**FIG. 5** illustrates a flow diagram depicting an example method for determining an optimal calibration time for a continuous analyte sensor, according to some embodiments disclosed herein.
**FIG. 6A** illustrates a flow diagram depicting an example method for detecting and compensating for a compression artifact with a continuous analyte sensor, according to some embodiments disclosed herein.
**FIG. 6B** illustrates a flow diagram depicting an example method for detecting and compensating for a compression event, according to some embodiments disclosed herein.
**FIG. 6C** illustrates an example waveform indicating a compression event, according to some embodiments disclosed herein.
**FIG. 7A** illustrates a flow diagram depicting an example method for detecting and compensating for a dip and recover event with a continuous analyte sensor, according to some embodiments disclosed herein.
**FIG. 7B** schematically illustrates an example multi-analyte sensing mechanism of a continuous analyte sensor, according to some embodiments disclosed herein.
**FIG. 7C** illustrates a flow diagram depicting an example method for detecting and compensating for a dip and recover event, according to some embodiments disclosed herein.
**FIG. 7D** illustrates an example waveform indicating a dip and recover event, according to some embodiments disclosed herein.
**FIG. 8** illustrates a flow diagram depicting an example method for verifying a hypoglycemic event, according to some embodiments disclosed herein.
**FIG. 9** illustrates a flow diagram depicting an example method for detecting and compensating for a wear location of a continuous analyte sensor, according to some embodiments disclosed herein.
**FIG. 10** is a flow diagram depicting a method for training machine learning models for utilization with the methods of **FIGs. 4A-9****,** according to certain embodiments of the present disclosure.

To facilitate understanding, identical reference numerals have been used, where possible, to designate identical elements that are common to the figures. It is contemplated that elements disclosed in one aspect may be beneficially utilized on other aspects without specific recitation.

### DETAILED DESCRIPTION

Systems and methods according to the present principles relate to the monitoring of multiple analytes using a continuous multi-analyte monitoring and sensor system. More particularly, embodiments provided herein include improved systems and methods for: multiplexed sensing and data processing of multiple different analytes; calibration of multi-analyte sensors; detection and compensation for artifacts and related events; and detection and compensation for sensor location on the patient. Such systems and methods facilitate more accurate and reliable determinations of physiological states of a patient, including determinations and/or predictions of, e.g., glycemic events, and thus, enable better decision-making for management of disease, e.g., diabetes.

Accordingly, certain embodiments described herein provide a technical solution to the technical problems described above by providing a continuous multi-analyte monitoring system that is configured to monitor, generate, and analyze data for multiple analytes (e.g., glucose and, e.g., lactate) simultaneously and in a multiplexed manner. In certain embodiments, simultaneous monitoring of multiple analytes is enabled utilizing a device for multiplexed continuous analyte sensing, the device including one or more voxelated working electrodes, wherein each of the one or more voxelated working electrodes comprises a one or more enzymes deposited thereon. Each of the enzymes may be specific to a different analyte. Further, the device may include a reference electrode electrically coupled with each of the one or more voxelated working electrodes; a counter electrode electrically coupled with each of the one or more voxelated working electrodes; and a potentiostat electrically coupled with each of the one or more voxelated working electrodes, the potentiostat configured to switch a potential bias between the counter electrode and each of the one or more voxelated working electrodes for one or more predetermined time periods to detect the binding of one or more different analytes to the plurality of enzymes.

In certain embodiments, simultaneous monitoring of multiple analytes is enabled utilizing a device for multiplexed continuous analyte sensing, the device comprising: a working electrode, wherein the working electrode comprises an enzyme and an aptamer immobilized thereon, the enzyme and the aptamer each specific to a different analyte; a reference electrode electrically coupled to the working electrode with high impedance; a counter electrode electrically coupled to the working electrode with low impedance; a potentiostat electrically coupled to the working electrodes, reference electrode, and counter electrode, the potentiostat configured to apply a potential bias between the working electrode and counter electrode to determine a response current through the working electrode for detecting the enzyme catalysis and subsequent redox reactions; and an impedance analyzer electrically coupled to the working electrode, the impedance analyzer configured to determine impedance changes through the working electrode for detecting the binding of a corresponding analyte to the aptamer.

Certain embodiments described herein provide a technical solution to the problem described above by providing methods for automatic correction of one or more factory-calibrated continuous multi-analyte sensors. In certain embodiments, such methods include a method for calibrating a continuous analyte sensor, the method comprising: using a continuous analyte sensor, continuously monitoring a first analyte of a patient during a time period to obtain measured analyte data for the first analyte; continuously monitoring other measured sensor data indicative of a physiological state of the patient during the time period; processing the measured sensor data from the time period to determine expected analyte data for the first analyte based on the physiological state of the patient during the time period; determining a compensation factor between the expected analyte data and the measured analyte data, the compensation factor indicative of an error in calibration of the continuous analyte sensor; and determining whether recalibration of the continuous analyte sensor is possible, wherein: if recalibration is possible, recalibrating the continuous analyte sensor based on the compensation factor, or if recalibration is not possible, recommending, to the patient, to replace the continuous analyte sensor.

In certain embodiments, such methods for automatic correction of one or more factory-calibrated continuous multi-analyte sensors include a method for calibrating a continuous analyte sensor, the method comprising: receiving data indicative of a historical relationship between a first analyte and a second analyte of a patient during a first time period; using at least one continuous analyte sensor, continuously monitoring the first analyte of the patient during a second time period to obtain measured first analyte data; using the at least one continuous analyte sensor, continuously monitoring the second analyte of the patient during the second time period to obtain measured second analyte data; processing the measured first and second analyte data for the second time period to determine a current relationship between the first analyte and the second analyte; and determining whether there is a substantial mismatch between the historical relationship and the current relationship, the substantial mismatch indicative of an error in calibration of the first and/or second continuous analyte sensor, wherein: if there is a substantial mismatch, recommending, to the patient, to recalibrate the first and/or the second continuous analyte sensor.

In certain embodiments, the first analyte is glucose and the second analyte is lactate.

In certain embodiments, the method for calibrating a continuous analyte sensor further comprises determining whether recalibration of the first or second continuous analyte sensor is possible, wherein: if recalibration is not possible, recommending, to the patient, to replace the first and/or the second continuous analyte sensor.

Certain embodiments described herein provide a technical solution to the technical problem described above by providing methods for determining an optimal calibration time for one or more continuous multi-analyte sensors. In certain embodiments, such methods include a method for determining when to recalibrate a continuous analyte sensor, the method comprising: continuously monitoring a first analyte of a patient during a time period to obtain measured first analyte data; determining a period of stability based on the measured first analyte data; and providing a recommendation for optimal calibration time based on the period of stability.

In certain embodiments, the method for determining when to recalibrate a continuous analyte sensor further comprises continuously monitoring a second analyte of the patient during the time period to obtain measured second analyte data, wherein the period of stability is further based on the measured second analyte data.

In certain embodiments, the method for determining when to recalibrate a continuous analyte sensor further comprises receiving measured physiological data indicative of a physiological state of the patient during the time period, wherein the period of stability is further based on the measured physiological data.

In certain embodiments, the period of stability has a duration of at least 5 minutes.

In certain embodiments, the optimal calibration time correlates with a time that a measurement of the first analyte is taken.

In certain embodiments, the optimal calibration time correlates with a time that a measurement of the first analyte is displayed for a user.

Certain embodiments described herein provide a technical solution to the technical problem described above by providing methods for detecting and compensating for compression events. In certain embodiments, such methods include a method for continuous analyte sensing, the method comprising: continuously monitoring a first analyte of a patient during a time period to obtain measured first analyte data; continuously monitoring one or more second analytes of the patient during the time period to obtain measured second analyte data; receiving measured physiological data indicating a physiological state of the patient; and determining whether a compression event has occurred based on the measured first and second analyte data and the physiological state of the patient.

In certain embodiments, the method for continuous analyte sensing further comprises: detecting a change in first analyte levels that is greater than a predetermined normal threshold; and determining whether the patient was resting or engaging in physical activity during the change in first analyte levels, wherein: if the patient was resting, determining whether the patient changed posture prior to the change in first analyte levels; and if the patient was engaging in physical activity, determining whether there was a detected change in lactate levels corresponding to the change in first analyte levels.

In certain embodiments, such methods for detecting and compensating for compression events comprise a method for calibrating a continuous analyte sensor, the method comprising: using a continuous analyte sensor, continuously monitoring glucose of a patient during a time period to obtain measured glucose data; continuously monitoring lactate of the patient during the time period to obtain measured lactate data; determining that a decrease in glucose levels that is greater than a predetermined normal threshold; determining whether there is a detected increase in lactate levels corresponding to the decrease in glucose levels, wherein: if there is a detected corresponding increase in lactate levels, recalibrating the continuous analyte sensor based on the measured lactate data.

In certain embodiments, such methods for detecting and compensating for compression events comprise a method for calibrating a continuous analyte sensor, the method comprising: using a continuous analyte sensor, continuously monitoring glucose of a patient during a time period to obtain measured glucose data; continuously monitoring lactate of the patient during the time period to obtain measured lactate data; determining that a decrease in glucose levels that is greater than a predetermined normal threshold; determining whether there is a detected increase in lactate levels corresponding to the decrease in glucose levels, wherein: if there is a detected corresponding increase in lactate levels, providing an alert or notification to the user that a compression event is detected.

Certain embodiments described herein provide a technical solution to the technical problem described above by providing methods for determining a wear location of a continuous analyte sensor. In certain embodiments, such methods include a method for continuous analyte sensing, the method comprising: using a first continuous analyte sensor, continuously monitoring a first analyte of a patient during a time period to obtain measured first analyte data; receiving measured physiological data indicating a physiological state of the patient during the time period; determining a responsiveness of the first analyte to physiological events of the patient based on the measured first analyte data and the physiological data; determining a wear location of the first continuous analyte sensor based on the responsiveness of the first analyte; and recalibrating the first continuous analyte sensor based on the detected wear location.

In certain embodiments described herein, a continuous multi-analyte monitoring system may be configured to collect a variety of data, including analyte data for multiple analytes, as well as patient information, secondary sensor data (e.g., non-analyte data), patient input, etc. For example, the analyte data may include continuously monitored glucose data, as well as continuously monitored data for lactate, ketones, glycerol, electrolytes such as sodium and potassium, calculated measurements such as anion gap, and other suitable analytes.

In certain embodiments described herein, methods for calibration and/or compensation of a continuous multi-analyte monitoring system are based on the collected analyte data for multiple analytes, in addition to other types of collected data. Such collected data may include secondary sensor data, such as accelerometer data, gyrometer data, global positioning system (GPS) data, heart rate, heart rate reserve, heart rate variability (HRV), electrocardiogram (EKG) data, electromyogram (EMG), respiration rate, temperature, blood pressure, galvanic skin response, oxygen uptake data (e.g., VO₂ max), sleep, impedance data, strain sensor data, etc. Such collected data may further include patient information, such as age, gender, glucose threshold levels, previous exercise-related or nutrition related event data, fitness levels, activity frequency, etc.

In certain embodiments described herein, a continuous multi-analyte monitoring system may use various algorithms or artificial intelligence (AI) models, such as machine-learning models, trained based on patient-specific data and/or population data to facilitate calibration and/or compensation (e.g., for artifacts or wear location) based on collected information about a patient. For example, the algorithms and/or machine-learning models may take into account parameters, such as analyte levels of the patient, in addition to secondary sensor data and/or patient information (e.g., personalized patient-specific data), during calibration and/or compensation of a continuous multi-analyte monitoring system. Based on these parameters, the algorithms and/or machine-learning models may facilitate improved calibration and/or compensation.

According to certain embodiments, prior to deployment, the machine learning models are trained with training data, e.g., including population data. As described herein, the population data may be provided in the form of a dataset including data records or samples of historical patients (e.g., with diabetes). Each data record is then featurized (e.g., refined into a set of one or more features, or predictor variables) and labeled. Data labeling is the process of adding one or more meaningful and informative labels to provide context to the data for learning by the machine learning models. The features associated with each data record may be used as input into the machine learning models, and the generated output may be compared to label(s) assigned to each of the data records. The models may compute a loss based on the difference between the generated output and the provided label(s). This loss can then be used to modify the internal parameters or weights of the models. By iteratively processing features associated with each data record corresponding to each historical patient, the models may be iteratively refined to facilitate improved calibration and/or compensation.

In certain embodiments, through the combination of a continuous multi-analyte monitoring system with machine learning models and/or algorithms for calibration and/or compensation (e.g., for artifacts or wear location), the systems and methods described herein may provide the necessary accuracy and reliability that diabetic patients expect from continuous analyte monitoring systems. For example, continuous monitoring of multiple analytes, e.g., glucose and lactate, may provide a more complete picture of a patient's physiological state, particularly when compared to utilizing a single analyte; similarly, utilizing machine learning models and/or algorithms for calibration and/or compensation may provide improved accuracy of continuous multi-analyte sensors as compared to traditional calibration methods. Accordingly, the improved systems and methods herein may facilitate more reliable determinations and/or predictions of, e.g., glycemic events of diabetic patients, and thus, enable better decision making for managing diabetes and/or other diseases.

The term "calibration," as used herein, is a broad term, and is used in its ordinary sense, and refers without limitation to a process of determining the relationship between raw sensor data to clinically meaningful units.

The term "calibrated data," as used herein, is a broad term, and is used in its ordinary sense, and refers without limitation to data that has been transformed from its raw state to another state using a function or series of functions, lookup table, etc., to provide a clinically meaningful value to a user.

The term "continuous," as used herein, is a broad term, and is used in its ordinary sense, and can mean continuous, semi-continuous, continual, periodic, intermittent, regular, etc.

The terms "continuous analyte sensor," "continuous multi-analyte sensor," "continuous glucose sensor," and "continuous lactate sensor," as used herein, are broad terms, and are used in their ordinary sense, and refer without limitation to a device that continuously measures a concentration of an analyte and/or calibrates the device (e.g., by continuously adjusting or determining the sensor's sensitivity and background), for example, at time intervals ranging from fractions of a second up to, e.g., 1, 2, or 5 minutes, or longer.

The terms "sensitivity" or "sensor sensitivity," as used herein, are broad terms, and are used in their ordinary sense, and refer without limitation to an amount of signal produced by a certain concentration of a measured analyte, or a measured species (e.g., H₂O₂) associated with a measured analyte (e.g., glucose or lactate). For example, a sensor may have a sensitivity of from about 1 to about 300 picoAmps of current for every 1 mg/dL of glucose analyte.

The term "sensor data," as used herein, is a broad term, and is used in its ordinary sense, and refers without limitation to any data associated with a sensor, such as a continuous analyte or continuous multi-analyte sensor. Sensor data includes a raw data stream, or simply data stream, of analog or digital signal directly related to a measured analyte from an analyte sensor (or other signal received from another sensor), as well as calibrated and/or filtered raw data. The terms "sensor data point" and "data point" refer generally to a digital representation of sensor data at a particular time. The terms broadly encompass a plurality of time spaced data points from a sensor, such as a continuous analyte sensor, which comprises individual measurements taken at time intervals ranging from fractions of a second up to, e.g., 1, 2, or 5 minutes or longer. In another example, the sensor data includes an integrated digital value representative of one or more data points averaged over a time period. Sensor data may include calibrated data, smoothed data, filtered data, transformed data, and/or any other data associated with a sensor.

The term "sensor electronics," as used herein, is a broad term, and is used in its ordinary sense, and refers without limitation to components, e.g., hardware and/or software, of a device configured to process sensor data.

Although certain embodiments herein are described with reference to management of diabetes, diabetes management is only an example of one application for which the present systems and methods may be utilized. The systems and methods described herein can also be used for managing one or more other diseases or conditions, which may or may not include diabetes. For example, the systems and methods described herein can be utilized for managing kidney disease, liver disease, and other types of diseases or conditions.

### Example Disease Management System Including an Example Continuous Multi-Analyte Monitoring System

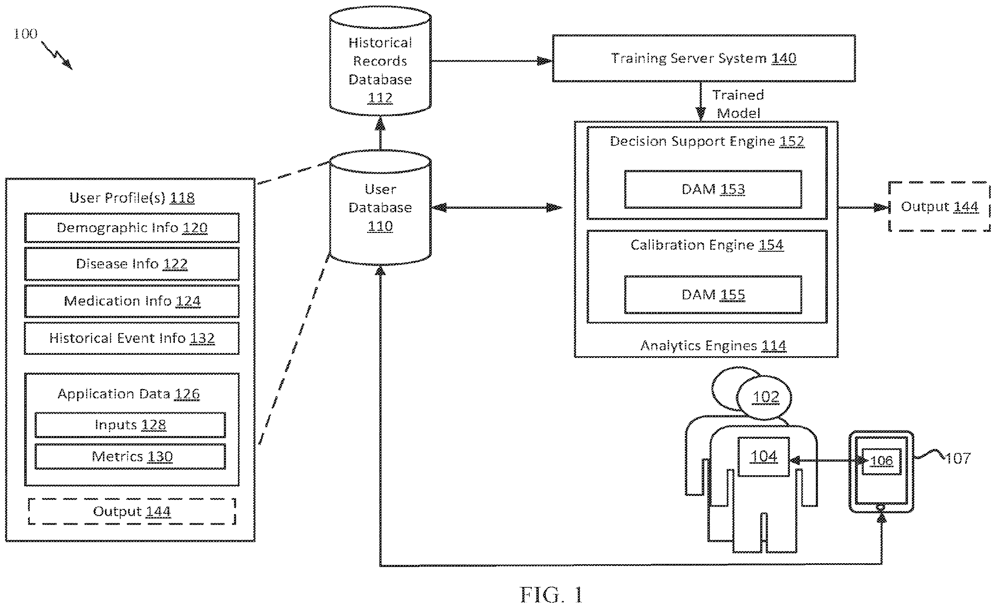

**FIG. 1** illustrates example disease management system 100 for assisting users 102 (individually referred to herein as a user and collectively referred to herein as users) with decision support for managing a disease, e.g., diabetes, kidney disease, liver disease, and/or other types of diseases. The system 100 utilizes continuous analyte monitoring system 104 configured to continuously measure one or more of a plurality of analytes, such as glucose, lactate, potassium, creatinine, ketone, alcohol, etc. A user, in certain embodiments, may be a patient or, in some cases, the patient's caregiver. In certain embodiments, system 100 includes continuous analyte monitoring system 104, a display device 107 that executes application 106, analytics engines 114, a user database 110, a historical records database 112, and a training server system 140, each of which is described in more detail below.

The term "analyte" as used herein is a broad term used in its ordinary sense, including, without limitation, to refer to a substance or chemical constituent in a biological fluid (for example, blood, interstitial fluid, cerebral spinal fluid, lymph fluid, sweat, or urine) that can be analyzed. Analytes can include naturally occurring substances, artificial substances, metabolites, and/or reaction products. Analytes for measurement by the devices and methods may include, but may not be limited to, glucose, acarboxyprothrombin; acylcarnitine; adenine phosphoribosyl transferase; adenosine deaminase; albumin; alpha-fetoprotein; amino acid profiles (arginine (Krebs cycle), histidine/urocanic acid, homocysteine, phenylalanine/tyrosine, tryptophan); andrenostenedione; antipyrine; arabinitol enantiomers; arginase; benzoylecgonine (cocaine); biotinidase; biopterin; c-reactive protein; carnitine; carnosinase; CD4; ceruloplasmin; chenodeoxycholic acid; chloroquine; cholesterol; cholinesterase; conjugated 1-β hydroxy-cholic acid; cortisol; creatine kinase; creatine kinase MM isoenzyme; cyclosporin A; d-penicillamine; de-ethylchloroquine; dehydroepiandrosterone sulfate; DNA (acetylator polymorphism, alcohol dehydrogenase, alpha 1-antitrypsin, cystic fibrosis, Duchenne/Becker muscular dystrophy, glucose-6-phosphate dehydrogenase, hemoglobin A, hemoglobin S, hemoglobin C, hemoglobin D, hemoglobin E, hemoglobin F, D-Punjab, beta-thalassemia, hepatitis B virus, HCMV, HIV-1, HTLV-1, Leber hereditary optic neuropathy, MCAD, RNA, PKU, Plasmodium vivax, sexual differentiation, 21-deoxycortisol); desbutylhalofantrine; dihydropteridine reductase; diptheria/tetanus antitoxin; erythrocyte arginase; erythrocyte protoporphyrin; esterase D; fatty acids/acylglycines; free β-human chorionic gonadotropin; free erythrocyte porphyrin; free thyroxine (FT4); free tri-iodothyronine (FT3); fumarylacetoacetase; galactose/gal-1-phosphate; galactose-1-phosphate uridyltransferase; gentamicin; glucose-6-phosphate dehydrogenase; glutathione; glutathione perioxidase; glycocholic acid; glycerol; glycosylated hemoglobin; halofantrine; hemoglobin variants; hexosaminidase A; human erythrocyte carbonic anhydrase I; 17-alpha-hydroxyprogesterone; hypoxanthine phosphoribosyl transferase; immunoreactive trypsin; lactate; lead; lipoproteins ((a), B/A-1, β); lysozyme; mefloquine; netilmicin; phenobarbitone; phenytoin; phytanic/pristanic acid; progesterone; prolactin; prolidase; purine nucleoside phosphorylase; potassium, quinine; reverse tri-iodothyronine (rT3); selenium; serum pancreatic lipase; sissomicin; somatomedin C; specific antibodies (adenovirus, anti-nuclear antibody, anti-zeta antibody, arbovirus, Aujeszky's disease virus, dengue virus, Dracunculus medinensis, Echinococcus granulosus, Entamoeba histolytica, enterovirus, Giardia duodenalisa, Helicobacter pylori, hepatitis B virus, herpes virus, HIV-1, IgE (atopic disease), influenza virus, Leishmania donovani, leptospira, measles/mumps/rubella, Mycobacterium leprae, Mycoplasma pneumoniae, Myoglobin, Onchocerca volvulus, parainfluenza virus, Plasmodium falciparum, poliovirus, Pseudomonas aeruginosa, respiratory syncytial virus, rickettsia (scrub typhus), Schistosoma mansoni, Toxoplasma gondii, Trepenoma pallidium, Trypanosoma cruzi/rangeli, vesicular stomatis virus, Wuchereria bancrofti, yellow fever virus); specific antigens (hepatitis B virus, HIV-1); succinylacetone; sulfadoxine; theophylline; thyrotropin (TSH); thyroxine (T4); thyroxine-binding globulin; trace elements; transferrin; UDP-galactose-4-epimerase; urea; uroporphyrinogen I synthase; vitamin A; white blood cells; and zinc protoporphyrin.

Salts, sugar, protein, fat, vitamins, and hormones naturally occurring in blood or interstitial fluids can also constitute analytes in certain implementations. The analyte can be naturally present in the biological fluid, for example, a metabolic product, a hormone, an antigen, an antibody, and the like. Alternatively, the analyte can be introduced into the body or exogenous, for example, a contrast agent for imaging, a radioisotope, a chemical agent, a fluorocarbon-based synthetic blood, or a drug or pharmaceutical composition, including but not limited to insulin; glucagon, ethanol; cannabis (marijuana, tetrahydrocannabinol, hashish); inhalants (nitrous oxide, amyl nitrite, butyl nitrite, chlorohydrocarbons, hydrocarbons); cocaine (crack cocaine); stimulants (amphetamines, methamphetamines, Ritalin, Cylert, Preludin, Didrex, PreState, Voranil, Sandrex, Plegine); depressants (barbiturates, methaqualone, tranquilizers such as Valium, Librium, Miltown, Serax, Equanil, Tranxene); hallucinogens (phencyclidine, lysergic acid, mescaline, peyote, psilocybin); narcotics (heroin, codeine, morphine, opium, meperidine, Percocet, Percodan, Tussionex, Fentanyl, Darvon, Talwin, Lomotil); designer drugs (analogs of fentanyl, meperidine, amphetamines, methamphetamines, and phencyclidine, for example, Ecstasy); anabolic steroids; and nicotine. The metabolic products of drugs and pharmaceutical compositions are also contemplated analytes. Analytes such as neurochemicals and other chemicals generated within the body can also be analyzed, such as, for example, ascorbic acid, uric acid, dopamine, noradrenaline, 3-methoxytyramine (3MT), 3,4-Dihydroxyphenylacetic acid (DOPAC), Homovanillic acid (HVA), 5-Hydroxytryptamine (5HT), and 5-Hydroxyindoleacetic acid (FHIAA), and intermediaries in the Citric Acid Cycle.

In certain embodiments, the analytes that are measured and analyzed by the devices and methods described herein include glucose, lactate, ketones, potassium, and in some examples, other analytes listed above. However, other analytes, which are not listed above, may also be considered.

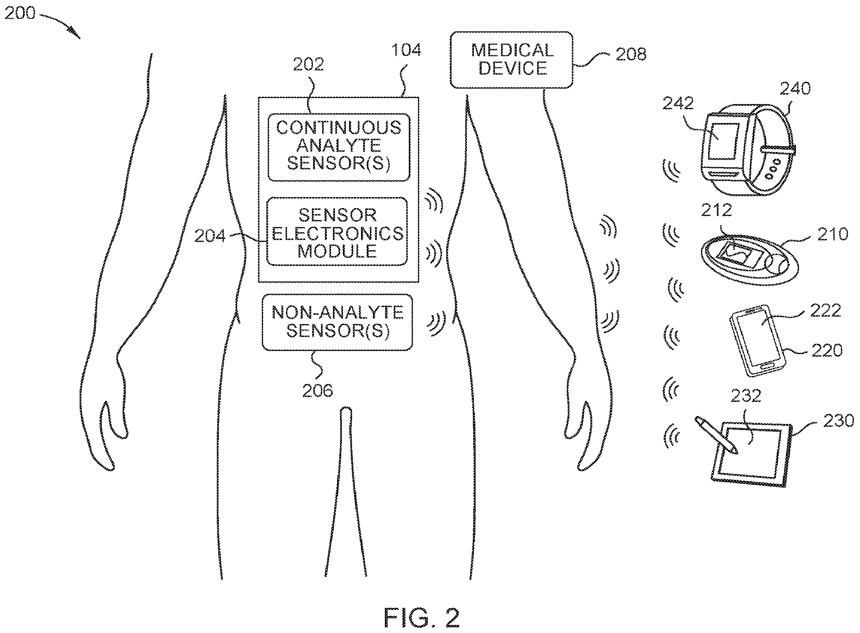

In certain embodiments, continuous analyte monitoring system 104 is configured to continuously measure one or more analytes and transmit the analyte measurements to display device 107 for use by application 106. In some embodiments, continuous analyte monitoring system 104 transmits the analyte measurements to display device 107 through a wireless connection (e.g., Bluetooth connection). In certain embodiments, display device 107 is a smart phone. However, in certain other embodiments, display device 107 may instead be any other type of computing device such as a laptop computer, a smart watch, a fitness tracker, a tablet, or any other computing device capable of executing application 106. Continuous analyte monitoring system 104 may be described in more detail with respect to **FIG. 2****.**

Application 106 is a mobile health application that is configured to receive and analyze analyte measurements from analyte monitoring system 104. For example, application 106 stores information about a user, including the user's analyte measurements, in a user profile 118 of the user for processing and analysis as well as for use by the decision support engine 152 to provide decision support recommendations or guidance to the user.

Analytics engines 114 include decision support engine 152 and calibration engine 154. In certain embodiments, decision support engine 152 and calibration engine 154 may be implemented as a set of software instructions. As discussed in more detail herein, decision support engine 152 may provide disease management decision support recommendations to the user, e.g., via application 105, and calibration engine 154 may drive calibration and/or calibration-related processes of one or more sensors of continuous analyte monitoring system 104. Decision support engine 152 may provide such recommendations, and/or calibration engine 154 may drive such calibrations, based on analyte measurements associated with one or more analytes received from continuous analyte monitoring system 104, data obtained from one or more non-analyte sensors or devices, and/or information included in user profile 118. In certain embodiments, analytics engines 114 execute entirely on one or more computing devices in a private or a public cloud. In such embodiments, application 106 communicates with analytics engines 114 over a network (e.g., Internet). In some other embodiments, analytics engines 114 execute partially on one or more local devices, such as display device 107 or a processor associated with the continuous analyte monitoring system 104, and partially on one or more computing devices in a private or a public cloud. In some other embodiments, analytics engines 114 execute entirely on one or more local devices, such as display device 107 or a processor associated with the continuous analyte monitoring system 104.

User profile 118 may include information collected about the user from application 106. For example, application 106 provides a set of inputs 128, including the analyte measurements associated with one or more analytes received from continuous analyte monitoring system 104 that are stored in user profile 118. In certain embodiments, inputs 128 provided by application 106 include other data in addition to analyte measurements. For example, application 106 may obtain additional inputs 128 through manual user input, one or more other non-analyte sensors or devices (e.g., temperature sensors, etc.), other applications executing on display device 107, etc. Non-analyte sensors and devices include one or more of, but are not limited to, an insulin pump, respiratory sensor, sensors or devices provided by display device 107 (e.g., accelerometer, gyrometer, camera, global positioning system (GPS), heart rate monitor, etc.) or other user accessories (e.g., a smart watch), or any other sensors or devices that provide relevant information about the user. Inputs 128 of user profile 118 provided by application 106 may, for example, include continuous analyte sensor data, non-analyte sensor data, time, food consumption, physical activity, sleep information, user statistics, medication, etc.

In the embodiments of FIG. 1, each of decision support engine 152 and calibration engine 154 include a data analysis module (DAM) 153 or 155, respectively. Note that in other embodiments, however, decision support engine 152 and calibration engine 154 may share a single DAM. In certain embodiments, the DAM(s) are configured to process one or more inputs 128 and determine one or more metrics 130. Metrics 130 may, at least in some cases, be generally indicative of the health or state of a user, e.g., the physiological state of a user. Examples of metrics 130 include glucose levels, glucose thresholds, glucose trends, lactate levels, lactate baseline, lactate threshold, lactate trends, ketone levels, ketone production rates, insulin sensitivity, insulin on board, meal state, meal habits, physical fitness, metabolic rate, body temperature, blood pressure, heart rate, heart rate variability, respiratory rate, blood-alcohol concentration, etc. In certain embodiments, metrics 130, in addition to inputs 128, may then be used by decision support engine 152 and/or calibration engine 154 as input for performing decision support or calibrating one or more continuous multi-analyte sensors, respectively. As shown, metrics 130 may also be stored in user profile 118.

User profile 118 further includes demographic info 120, disease info 122, and/or medication info 124. In certain embodiments, such information may be provided through user input or obtained from certain data stores (e.g., electronic medical records, etc.). In certain embodiments, demographic info 120 may include one or more of the user's age, BMI (body mass index), ethnicity, gender, etc. In certain embodiments, disease info 122 may include information about one or more diseases of a user, including relevant information pertaining to the user's condition of diabetes and/or other conditions (e.g., liver disease, kidney disease, etc.). In certain embodiments, disease info 122 may also include the length of time since diagnosis, the level of disease control, level of compliance with disease management therapy, other types of diagnoses (e.g., heart disease, obesity), etc. In certain embodiments, disease info 122 may include other measures of health (e.g., heart rate, stress, sleep, etc.) or fitness (e.g., cardiovascular endurance, muscular strength and/or power, muscular endurance, and other measures of fitness), and/or the like.

In certain embodiments, medication info 124 may include information about the amount and type of a medication taken by a user. In certain embodiments, medication information may include information about the consumption of one or more drugs for management of the user's condition of diabetes, such as insulin (e.g., short-acting insulin, rapid-acting insulin (insulin aspart, insulin gluilisine, insulin lispro), intermediate-acting insulin (insulin isophane), long-acting insulin degludec, indulin detemir, insulin glargine, insulin), combination insulins), amylinomimetic drugs, alpha-glucosidase inhibitors (e.g., acarbose, miglitol), biguanides (e.g., metformin-alogliptin, metformin-canagliflozin, metformin-dapagliflozin, metformin-empagliflozin, metformin-glipizide, metformin-glyburide, metformin-linagliptin, metformin-pioglitazone, metformin-repaglinide, metformin-rosiglitazone, metformin-saxagliptin, metformin-sitagliptin), dopamine agonists (e.g., bromocriptine), dipeptidyl peptidase-4 (DPP-4) inhibitors (e.g., alogliptin, alogliptin-pioglitazone, linagliptin, linagliptin-empagliflozin, saxagliptin, sitagliptin, simvastatin), glucagon-like peptide-1 receptor agonists (GLP-1 receptor agonists) (e.g., albiglutide, dulaglutide, exenatide, liraglutide, semaglutide), meglitinides (e.g., nateglinide, repaglinide), sodium-glucose transporter (SGLT) 2 inhibitors (e.g., dapagliflozin, canagliflozin, empagliflozin, ertugliflozin), sulfonylureas (e.g., glimepiride, glimepiride-pioglitazone, glimepiride-rosiglitazone, gliclazide, glipizide, glyburide, chlorpropamide, tolazamide, tolbutamide), thiazolidinediones (e.g., rosiglitazone, pioglitazone), and other drugs. In certain embodiments, medication information may include information about the consumption of one or more drugs for management or treatment of other diseases or conditions of the user, including drugs for cholesterol, high blood pressure, heart disease, etc., in addition to supplements to promote general health and metabolism, such as vitamins.

Data stored in user profile 118 may maintain time series data collected for the user (e.g., the patient) over a period of time that the user utilizes continuous analyte monitoring system 104 and application 106. For example, analyte data for a user who has used continuous analyte monitoring system 104 and application 106 for a period of five years to manage their condition may have time series analyte data associated with the user maintained in user profile 118 over the five-year period.

Further, data stored in user profile 118 may provide time series data collected over the lifetime of the user. For example, the data may include information associated with the user that indicates physiological states of the user, glucose levels of the user, lactate levels of the user, ketone levels of user, states/conditions of one or more organs of the user, habits of the user (e.g., alcohol consumption, activity levels, food consumption, etc.), medication(s) prescribed, etc., throughout the lifetime of the user.

In certain embodiments, user profile 118 is dynamic because at least part of the information that is stored in user profile 118 may be revised or updated over time and/or new information may be added to user profile 118 by analytics engines 114 and/or application 106. Accordingly, information in user profile 118 stored in user database 110 provides an up-to-date repository of information related to the user.

User database 110, in some embodiments, refers to a storage server that operates, for example, in a public or private cloud. User database 110 may be implemented as any type of datastore, such as relational databases, non-relational databases, key-value datastores, file systems including hierarchical file systems, and the like. In some exemplary implementations, user database 110 is distributed. For example, user database 110 may comprise a plurality of persistent storage devices, which are distributed. Furthermore, user database 110 may be replicated so that the storage devices are geographically dispersed.

User database 110 includes user profiles 118 associated with a plurality of users, including users who similarly interact or have interacted in the past with application 106 on their own devices. User profiles stored in user database 110 are accessible to not only to application 106, but to analytics engines 114, as well. User profiles in user database 110 may be accessible to application 106 and analytics engines 114 over one or more networks (not shown). As described above, analytics engines 114, and more specifically, data analysis modules 153, 155 of analytics engines 114, can fetch inputs 128 from a user's profile 118 stored in user database 110 and compute one or more metrics 130, which can then be stored as application data 126 in the user's profile 118.

In certain embodiments, user profiles 118 stored in user database 110 may also be stored in historical records database 112. User profiles 118 stored in historical records database 112 may provide a repository of up-to-date information and historical information for each user of application 106. Thus, historical records database 112 essentially provides all data related to each user of application 106, where data is stored using timestamps. The timestamp associated with any piece of information stored in historical records database 112 may identify, for example, when the piece of information was obtained and/or updated.

Further, in certain embodiments, historical records database 112 may include data for one or more patients who are not users of continuous analyte monitoring system 104 and/or application 106. For example, historical records database 112 may include information (e.g., user profile(s)) related to one or more patients analyzed by, for example, a healthcare physician (or other known method), who may or may not be diagnosed with diabetes. Data stored in historical records database 112 may be referred to herein as population data.

Although depicted as separate databases for conceptual clarity, in some embodiments, user database 110 and historical records database 112 may operate as a single database. The single database may be a storage server that operates in a public or private cloud.

In addition to decision support, disease management system 100 is configured to perform various determinations with regard to recalibration, as well as detection and compensation of artifacts and other physiological events, using continuous analyte monitoring system 104 configured to continuously measure one or more analytes. For example, in certain embodiments, calibration engine 154 is configured to provide real-time or retrospective calibration-related determinations, thus enabling immediate and automatic recalibration or compensation for faulty measurements, and preventing or reducing the occurrence of erroneous glycemic event predictions or treatment recommendations by decision support engine 152. In particular, calibration engine 154 may be used to collect information associated with a user in user profile 118 via DAM 155, to perform analytics thereon, and to use such analytics in making determinations with regard to recalibration or sensor measurement compensation. User profile 118 may be accessible to calibration engine 154 over one or more networks (not shown) for performing such analytics.

In certain embodiments, calibration engine 154 may utilize one or more trained machine learning models capable of performing analytics on information that calibration engine 154 has collected/received from user profile 118. In the illustrated embodiment of **FIG. 1****,** calibration engine 154 may utilize one or more different trained machine learning models provided by a training server system 140 for different types of calibration-related determinations. Although depicted as a separate server for conceptual clarity, in some embodiments, training server system 140 and analytics engines 114 may operate as a single server or system. That is, the one or more models may be trained and used by a single server, or may be trained by one or more servers and deployed for use on one or more other servers or systems.

Training server system 140 is configured to train the one or more machine learning models using training data, which may include data (e.g., from user profiles) associated with one or more patients (e.g., users or non-users of continuous analyte monitoring system 104 and/or application 106, e.g., diabetic patients). The training data may be stored in historical records database 112 and may be accessible to training server system 140 over one or more networks (not shown) for training the machine learning model(s).

The training data refers to a dataset that has been featurized and labeled. For example, the dataset may include a plurality of data records, each including information corresponding to a different user profile stored in user database 110, where each data record is featurized and labeled. In machine learning and pattern recognition, a feature is an individual measurable property or characteristic. Generally, the features that best characterize the patterns in the data are selected to create predictive machine learning models. Data labeling is the process of adding one or more meaningful and informative labels to provide context to the data for learning by the machine learning models. As an illustrative example, each relevant characteristic or data point of a user, which is reflected in a corresponding data record, may be a feature used in training the machine learning model. Such features may include age, gender, normal glucose ranges, normal lactate ranges (e.g., lactate baseline and/or peak), normal ranges for other analytes and/or other secondary sensor data, time of and/or duration associated with a glycemic event, glucose information associated with a glycemic event (e.g., glucose levels associated with a hypo- or hyperglycemic events, etc.), treatments taken, glucose information subsequent to treatment, data associated with fitness levels (e.g., resting heart rate (RHR), minutes of activity per day, etc.), data associated with diet, data associated with sensor placement or positioning, etc. Such features may be utilized to categorize whether measured analyte ranges are outliers or within expected ranges for a given user profile 118, which in turn can be utilized for recommendation or other determinations related to calibration.

The one or more models are then trained by training server system 140 using the featurized and labeled training data. In particular, the features of each data record may be used as input into the machine learning model(s), and the generated output may be compared to label(s) associated with the corresponding data record. In certain embodiments, the model(s) may compute a loss based on the difference between the generated output and the provided label(s). This loss is then used to modify the internal parameters or weights of the model. By iteratively processing each data record corresponding to each historical patient, the model(s) may be iteratively refined to generate accurate calibration-related determinations.

As illustrated in **FIG. 1****,** training server system 140 deploys these trained model(s) to analytics engines 114 for use during runtime. For example, calibration engine 154 may obtain user profile 118 associated with a user and stored in user database 110, use information in user profile 118 as input into the trained model(s), and output a determination utilized for calibration or recalibration of a sensor (e.g., shown as output 144 in **FIG. 1**). In certain embodiments, output 144 generated by calibration engine 154 may be utilized to automatically recalibrate a continuous analyte sensor or adjust sensor measurements upon detection of an artifact or other physiological event. In certain embodiments, output 144 may be utilized to provide a recommendation to the user regarding recalibration. In further embodiments, output 144 may be utilized in further determinations by calibration engine 154, and may further be stored in user database 110 and utilized to train or re-train the trained model(s). In certain embodiments, output 144 generated by calibration engine 154 may be stored in user profile 118

**FIG. 2** is a diagram 200 conceptually illustrating an example continuous analyte monitoring system 104 including example continuous analyte sensor(s) with sensor electronics, in accordance with certain aspects of the present disclosure. For example, system 104 may be configured to continuously monitor one or more analytes of a user, in accordance with certain aspects of the present disclosure.

Continuous analyte monitoring system 104 in the illustrated embodiment includes sensor electronics module 204 and one or more continuous analyte sensors 202 (individually referred to herein as continuous analyte sensor 202 and collectively referred to herein as continuous analyte sensors 202) associated with sensor electronics module 204. Sensor electronics module 204 may be in wired or wireless communication (e.g., directly or indirectly) with one or more of display devices 210, 220, 230, and 240. In certain embodiments, sensor electronics module 204 may also be in wired or wireless communication (e.g., directly or indirectly) with one or more medical devices, such as medical devices 208 (individually referred to herein as medical device 208 and collectively referred to herein as medical devices 208), and/or one or more other non-analyte sensors 206 (individually referred to herein as non-analyte sensor 206 and collectively referred to herein as non-analyte sensor 206).

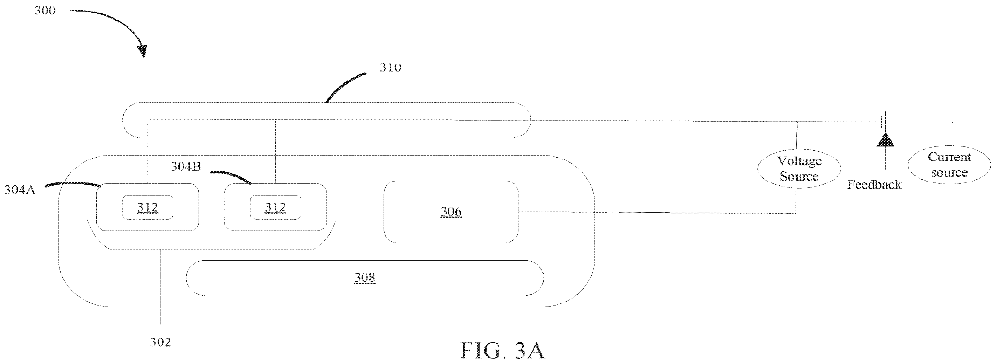

In certain embodiments, a continuous analyte sensor 202 may comprise one or more sensors for detecting and/or measuring analytes. A continuous analyte sensor 202 may be a multi-analyte sensor configured to continuously measure two or more analytes (e.g., glucose, lactate, potassium, ketone, etc.), or a single analyte sensor configured to continuously measure a single analyte (e.g., where one continuous analyte sensor 202 is used for measuring glucose and then a second continuous analyte sensor 202 used for measuring lactate, etc.). In certain embodiments, continuous analyte sensor 202 may be a non-invasive device, a subcutaneous device, a transcutaneous device, a transdermal device, and/or an intravascular device. In certain embodiments, the continuous analyte sensor 202 may be configured to continuously measure analyte levels of a user using one or more techniques, such as enzymatic techniques, chemical techniques, physical techniques, electrochemical techniques, spectrophotometric techniques, polarimetric techniques, calorimetric techniques, iontophoretic techniques, radiometric techniques, immunochemical techniques, and the like. The term "continuous," as used herein, can mean continuous, semi-continuous, continual, periodic, intermittent, regular, etc. Exemplary continuous sensing techniques are described with reference to **FIG. 3A-3B****.** In certain aspects, the continuous analyte sensor 202 provides a data stream indicative of the concentration of one or more analytes in the user. The data stream may include raw data signals, which are then converted into a calibrated and/or filtered data stream, e.g., by calibration engine 154 described above, and are used to provide estimated analyte value(s) to the user.

In certain embodiments, the continuous analyte sensor 202 may be a multi-analyte sensor, configured to continuously measure multiple analytes in a user's body. For example, in certain embodiments, the continuous multi-analyte sensor 202 may be a single sensor configured to measure glucose, lactate, ketones, glycerol, potassium (e.g., hyperkalemia), sodium, CO₂ or anion-gap, or similar analytes in the user's body.

In certain embodiments, sensor electronics module 204 includes electronic circuitry for measuring and processing the continuous analyte sensor data, including prospective algorithms associated with processing and calibration of the sensor data. Sensor electronics module 204 can be physically connected to continuous analyte sensor(s) 202 and can be integral with (non-releasably attached to) or releasably attachable to continuous analyte sensor(s) 202. Sensor electronics module 204 may include hardware, firmware, and/or software that enable measurement of levels of analyte(s) via continuous analyte sensor(s) 202. For example, sensor electronics module 204 can include a potentiostat, a power source for providing power to the sensor, other components useful for signal processing and data storage, and a telemetry module for transmitting data from the sensor electronics module to, e.g., one or more display devices. Electronics can be affixed to a printed circuit board (PCB), or the like, and can take a variety of forms. For example, the electronics can take the form of an integrated circuit (IC), such as an Application-Specific Integrated Circuit (ASIC), a microcontroller, and/or a processor. As another example, the sensor electronics module 204 may include a memory and a processor. The memory may store software instructions that the processor executes to perform any of the functions or actions of the continuous analyte monitoring system 104 described herein.

Display devices 210, 220, 230, and/or 240 are configured for displaying displayable sensor data, including analyte data, which may be transmitted by sensor electronics module 204. In one embodiment, the sensor electronics module 204 may transmit raw sensor data that is converted to displayable sensor data via one or more of display devices 210, 220, 230, and/04 240. In another embodiment, the sensor electronics module 204 may convert raw sensor data to displayable sensor data and transmit the displayable sensor data to the one or more of display devices 210, 220, 230, and/or 240. Each of display devices 210, 220, 230, or 240 may include a display such as a touchscreen display 212, 222, 232, and/or 242 for displaying sensor data to a user and/or for receiving inputs from the user. For example, a graphical user interface (GUI) may be presented to the user for such purposes. In some embodiments, the display devices may include other types of user interfaces such as a voice user interface instead of, or in addition to, a touchscreen display for communicating sensor data to the user of the display device and/or for receiving user inputs. Display devices 210, 220, 230, and 240 may be examples of display device 107 illustrated in **FIG. 1** used to display sensor data to a user of the system of **FIG. 1** and/or to receive input from the user.

In some embodiments, one, some, or all of the display devices are configured to display or otherwise communicate the sensor data as it is communicated from the sensor electronics module (e.g., in a customized data package that is transmitted to display devices based on their respective preferences), without any additional prospective processing required for calibration and real-time display of the sensor data.

The plurality of display devices may include a custom display device specially designed for displaying certain types of displayable sensor data associated with analyte data received from sensor electronics module. Display device 210 is an example of such a custom device. In some embodiments, one of the plurality of display devices is a smartphone, such as display device 220 which represents a mobile phone, using a commercially available operating system (OS), and configured to display a graphical representation of the continuous sensor data (e.g., including current and historic data). Other display devices can include other hand-held devices, such as display device 230 which represents a tablet, display device 240 which represents a smart watch, medical device 208 (e.g., an insulin delivery device or a blood glucose meter), and/or a desktop or laptop computer (not shown).

Because different display devices provide different user interfaces, content of the data packages (e.g., amount, format, and/or type of data to be displayed, alarms, and the like) can be customized (e.g., programmed differently by the manufacture and/or by an end user) for each particular display device. Accordingly, in certain embodiments, a plurality of different display devices can be in direct wireless communication with a sensor electronics module (e.g., such as an on-skin sensor electronics module 204 that is physically connected to continuous analyte sensor(s) 202) during a sensor session to enable a plurality of different types and/or levels of display and/or functionality associated with the displayable sensor information.

As mentioned, sensor electronics module 204 may be in communication with a medical device 208. Medical device 208 may be a passive device in some example embodiments of the disclosure. For example, medical device 208 may be an insulin pump for administering insulin to a user. For a variety of reasons, it may be desirable for such an insulin pump to receive and track analyte values, e.g., glucose values, transmitted from continuous analyte monitoring systems 104, where continuous analyte sensor 202 comprises at least a glucose sensor.

Further, as mentioned, sensor electronics module 204 may also be in communication with other non-analyte sensors 206. Non-analyte sensors 206 may include, but are not limited to, an altimeter sensor, an accelerometer sensor, a gyrometer sensor, a global positioning system (GPS) sensor, a temperature sensor, a respiratory sensor, electromyogram (EMG) sensor, a galvanic skin response (GSR) sensor, an impedance sensor, an electrocardiogram sensor, a sweat sensor, etc. Non-analyte sensors 206 may also include monitors such as heart rate monitors, blood pressure monitors, pulse oximeters, caloric intake monitors, and medicament delivery devices. One or more of these non-analyte sensors 206 may provide data to calibration engine 154 described further below. In some aspects, a user may manually provide some of the data for processing by training server system 140 and/or calibration engine 154 of **FIG. 1****.**

In certain embodiments, the non-analyte sensors 206 may be combined in any other configuration, such as, for example, combined with one or more continuous analyte sensors 202. As an illustrative example, a non-analyte sensor, e.g., a temperature sensor, may be combined with a continuous glucose sensor 202 to form a glucose/temperature sensor used to transmit sensor data to the sensor electronics module 204 using common communication circuitry. As another illustrative example, a non-analyte sensor, e.g., a temperature sensor, may be combined with a multi-analyte sensor 202 configured to measure glucose and, e.g., lactate to form a glucose/lactate/temperature sensor used to transmit sensor data to the sensor electronics module 204 using common communication circuitry.

One or more of continuous analyte monitoring system 104, the plurality of display devices, medical device(s) 208, and/or non-analyte sensor(s) 206 may be configured to communicate together wirelessly using one of a variety of wireless communication technologies (e.g., Wi-Fi, Bluetooth, Near Field Communication (NFC), cellular, etc.). In certain embodiments, a wireless access point (WAP) may be used to couple one or more of continuous analyte monitoring system 104, the plurality of display devices, medical device(s) 208, and/or non-analyte sensor(s) 206 to one another. For example, the WAP may provide Wi-Fi, Bluetooth, and/or cellular connectivity among these devices. NFC may also be used among devices depicted in diagram 200 of **FIG. 2****.**

### Example Continuous Multi-Analyte Sensors

**FIGs. 3A-3B** illustrate sensor electronics of example multi-analyte sensors 300 and 301, respectively, for use by the disease management system of **FIG. 1****,** according to some embodiments disclosed herein. In particular, multi-analyte sensors 300 and 301 are examples of continuous analyte sensor(s) 202 in **FIG. 2****.**

As shown in **FIG. 3A****,** multi-analyte sensor 300 is an enzyme-based "three-electrode" electrochemical sensor having a sensing region 302 comprising a plurality of working electrodes 304 (two working electrodes 304A and 304B are illustrated in **FIG. 3A**), reference electrode 306, and counter electrode 308, where, in certain aspects, the counter electrode 308 has a surface area that is greater (e.g., at least 10x) than that of the plurality of working electrodes 304 combined. Although only two working electrodes 304 are depicted, it is contemplated that multi-analyte sensor 300 may include any suitable number or working electrodes for multiplexed sensing of multiple analytes.

Electrodes 304, 306, and 308 may be formed of any suitable materials and by any suitable methods. For example, in certain embodiments, reference electrode 306 comprises silver (Ag) or silver/silver chloride (Ag/AgCl) and is kept currentless. In certain embodiments, counter electrode 308 is formed of, e.g., platinum (Pt), carbon (C), or other suitable inert materials. In certain embodiments, working electrodes 304 are formed of one or more noble metals, such as Pt or platinum/iridium (Pt/Ir). In other embodiments, working electrodes 304 are carbon-based, and comprise carbon (C) or carbon/ruthenium (C/Ru). In still further embodiments, working electrodes 304 are formed of graphite, gold, conductive polymer, or the like. Generally, suitable methods for forming electrodes 304, 306, and 308 include microfabrication techniques, such as physical vapor deposition, chemical vapor deposition, electrodeposition, lithography, and/or etching techniques. However, other methods, including spray deposition or dip-coating, are also contemplated.

Each of the plurality of working electrodes 304 comprises an active surface 312 to facilitate electrochemical sensing of desired analytes. In certain embodiments, the active surface 312 of each working electrode 304 is voxelated, or partitioned into discrete sections (e.g., cubic sections). In certain embodiments, a plurality of different enzymes (not shown), each specific to a different analyte, are deposited and immobilized on each active surface 312 of each working electrode 304 (e.g., each active surface 312 of each working electrode 304A and 304B in **FIG. 3A**). For example, in certain embodiments, both glucose oxidase and lactate or uric acid oxidase (and/or other analyte-specific enzymes) may be deposited on the active surface 312 of one working electrode 304A. In further embodiments, a plurality of different enzymes are immobilized on each voxel of each working electrode 304, while in other embodiments, only one type of enzyme is deposited on each voxel of each working electrodes 304. In certain embodiments, each of working electrodes 304 includes enzyme(s) for one specific analyte, while different working electrodes 304 may include enzymes for different analytes. In certain embodiments, one or more of each of working electrodes 304 comprises 2 or more enzymes, such as 3 or more enzymes, which together enable sensing of a single analyte. For example, in embodiments where a working electrode 304 is configured to sense creatinine, the working electrode 304 may comprise 3 or more enzymes specific for creatinine. An example of a 1-enzyme sensor working electrode 304 may include a lactate-specific electrode, and an example of a 2-enzyme sensor working electrode 304 may include a ketone-specific electrode. The enzymes may be immobilized via adsorption, entrapment, cross-linking, covalent bonding, or any other suitable immobilization methods.

During sensing, the deposited enzymes are utilized to convert a respective analyte to an intermediary product (e.g., hydrogen peroxide), which is then oxidized at the surface of the working electrodes 304. The resulting current flow, which is measured by potentiostat 310 or an ammeter in communication with potentiostat 310 (described below), is proportional to the analyte concentration. Examples of suitable enzymes include glucose oxidase for sensing glucose species, lactate oxidase for sensing lactate species, lactose oxidase for sensing lactose species, glutamate oxidase for glutamate species, and the like. In certain embodiments, in addition to enzymes, active surfaces 312 further include immobilized redox mediators (e.g., relays) (not shown), which are small electroactive molecules for shuttling electrons between the enzymes and the working electrodes 304. In certain embodiments, the enzymes are immobilized exclusively over the skive regions of working electrodes 304 to minimize or avoid cross-talk between different analytes. In other words, an area of active surface 312 over each of working electrodes 304 is less than a geometric surface area of the respective working electrode. In such embodiments, maintaining a potential bias at working electrodes 304 facilitates a near-zero peroxide efflux from the skive region with active consumption of the hydrogen peroxide intermediary.

As further shown, multi-analyte sensor 300 comprises potentiostat 310, which applies a potential to working electrodes 304 and reference electrode 306 and, in certain embodiments, measures a current value at each of the working electrodes. In certain embodiments, potentiostat 310 is communicatively coupled to an ammeter (not shown) for measuring the current value at each of the working electrodes. Potentiostat 310 comprises an integrated multiplexer interface to isolate signals from each of working electrodes 304, thus facilitating measurements from individual working electrodes without the need for a plurality of dedicated "single-channel" potentiostats. For example, in certain embodiments, potentiostat 310 is configured to switch potential bias between different working electrodes 304 for predetermined time periods or sampling frequencies. For example, potentiostat 310 may provide a potential to working electrode 304A for a predetermined time period of 1 second, 5 seconds, 10 seconds, 1 minute, 2 minutes, 3 minutes, 4 minutes, 5 minutes, 10 minutes, or more, before switching and providing a potential to working electrode 304B, and so on, to collect response currents for each working electrode 304. Response current measurements may be taken via various suitable methods, including differential pulse voltammetry (DPV), linear voltage sweep, or other voltammetric sweep methods, with different voltages applied to each working electrode 304 to accommodate different redox couples of different analytes.

In certain embodiments, to sense multiple analytes via a single working electrode 304, potentiostat 310 is configured to enable/disable and/or switch potential waveforms (e.g., shape, frequency, etc.) applied to the working electrodes 304 with mixed impedimetric and amperometric modalities. For example, in certain embodiments, potentiostat 310 may apply a first signal with a first waveform to a working electrode 304 during a first time period, and the impedance of the circuit may be measured to determine analyte levels of a first analyte. Thereafter, during a second time period, a second signal with a second waveform may be applied to the same working electrode 304, and the current through the circuit may be measured to determine analyte levels of a second analyte.

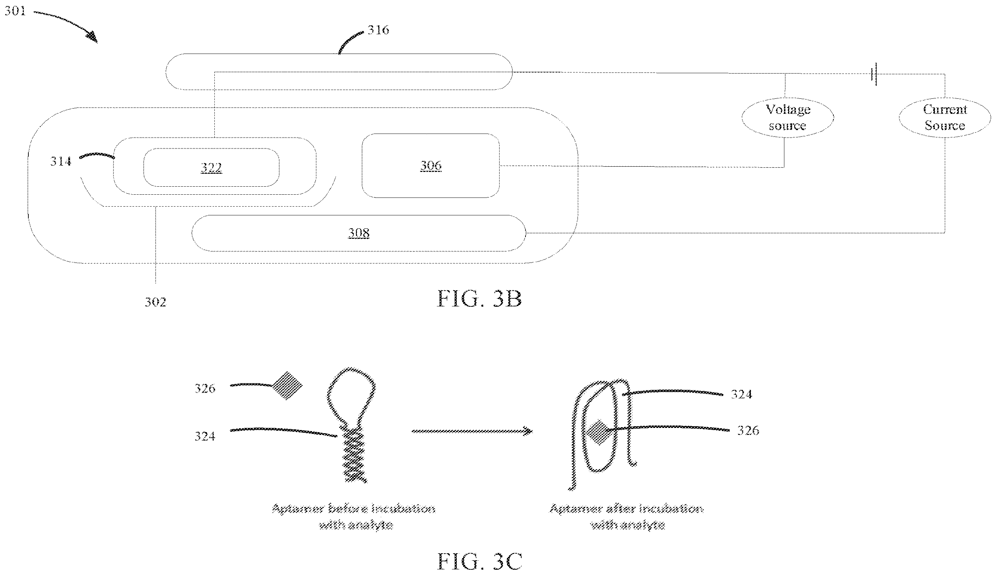

**FIG. 3B** illustrates sensor electronics of another example multi-analyte sensor 301, which comprises an enzyme- and aptamer-based "three-electrode" electrochemical sensor for detecting the presence of multiple desired analytes. Similar to multi-analyte sensor 300, multi-analyte sensor 301 includes reference electrode 306 and counter electrode 308, which may be formed of the same materials and by the same techniques as described above. In addition, multi-analyte sensor 301 further includes one or more working electrodes 314, as well as potentiostat 316, which may have an impedance analyzer (e.g., a broadband impedance analyzer) integrated or in communication therewith. Note that the electrode arrangements in the examples of **FIGs. 3A-3B** may, in certain embodiments, be utilized in combination. For example, in certain embodiments, a multi-analyte sensor may comprise a working electrode 304 of **FIG. 3A****,** as well as a working electrode 314 of **FIG. 3B****,** for detecting multiple different types of analytes.

Each of the one or more working electrodes 314 comprises an active surface 322 to facilitate electrochemical sensing of desired analytes. In certain embodiments, an enzyme (not shown) specific to an analyte is deposited and immobilized on the active surface 322. The enzyme may be immobilized via adsorption, entrapment, cross-linking, covalent bonding, or any other suitable immobilization methods. In certain embodiments, in addition to the enzyme, active surface 322 further includes an immobilized redox mediator (e.g., relay) (not shown), which facilitates electron transfer between the enzyme and the working electrode 314 to create a measurable current flow.

In certain embodiments, active surface(s) 322 further include one or more aptamers immobilized thereon. An example aptamer 324 and corresponding analyte 326 are depicted in **FIG. 3C** for reference. The aptamer 324 is a short, single stranded DNA or RNA molecule (e.g., ssDNA or ssRNA) that selectively binds to a specific analyte 326 (e.g., glucose, lactate, glutamate, etc.). In examples where the aptamer 324 is thiolated, the aptamer 324 may be immobilized on a gold active surface 322 via direct immobilization. However, other examples of suitable aptamer immobilization techniques that may be utilized with the systems and compositions herein include, but are not limited to: utilization of short linkers; streptavidin/biotin interactions; utilization of DNA nanostructures and a reduced graphene oxide as an immobilization platform; and/or physisorption.

Upon binding with analyte 326, aptamer 324 changes its secondary structure conformation, as depicted in **FIG. 3C****,** thereby causing a measurable change in impedance through working electrode 314 that can be measured by potentiostat 316, or an impedance analyzer (e.g., a broadband impedance analyzer, not shown) in communication therewith, using frequency sweep methods. The signal accurately represents the concentration of the bound analyte as along as enough aptamer is present on active surface(s) 322 in a concentration to last for a duration of sensor use or wear (e.g., enough available binding sites for accurate detection of analytes for the duration of sensor use). In certain embodiments, frequency sweeps can also be performed to monitor the sensor tissue interface for sources of noise, inaccuracy, etc. In embodiments where specific perturbation frequencies correlate strongly with analyte binding, an impedance analyzer may not be necessary. For example, in certain embodiments, impedance change can be determined by recording current in response to a time-varying voltage perturbation, though generally low in amplitude compared with most chronoamperometric applications.

Accordingly, the presence of both an enzyme, which can be specific to a first analyte, and an aptamer, which can be specific to a second analyte, on active surface 322 enables multi-analyte sensor 301 to detect multiple different analytes by switching waveforms between chronoamperometry methods to measure current flow through working electrode(s) 314, and frequency sweep detection methods to measure impedance through working electrode(s) 314. In other words, binding of the enzyme to a first analyte can be detected by measuring a magnitude of current through working electrode(s) 314, while binding of the aptamer to a second analyte can be detected by measuring a change in impedance through working electrode(s) 314. In certain embodiments, multi-analyte sensor 301 comprises multiple working electrodes 314, each having a different enzyme and a different aptamer immobilized thereon, and potentiostat 316 further comprises a multiplexer interface to isolate signals from each of working electrodes 314, thus facilitating isolation of measurements from individual working electrodes for multiplexed, multi-analyte sensing.

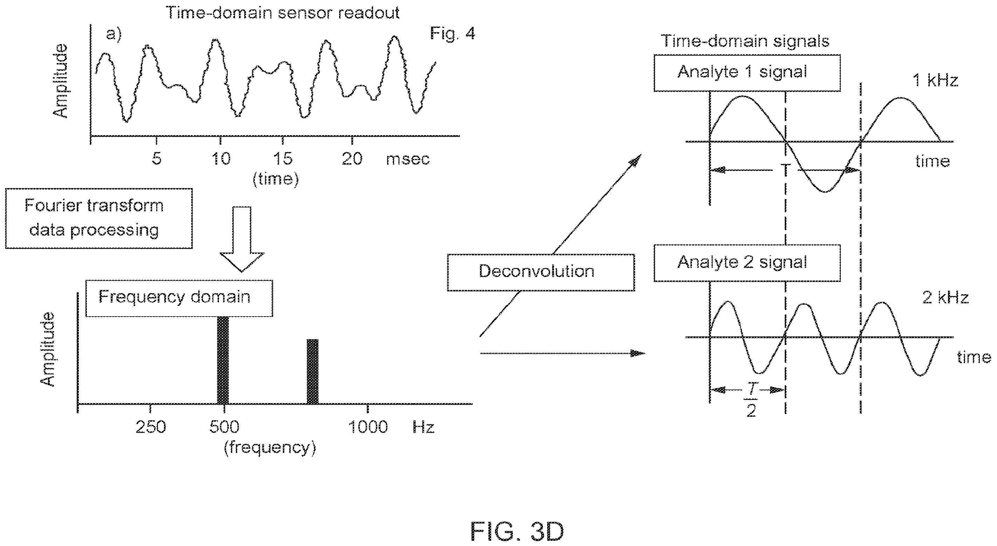

**FIG. 3D** graphically illustrates an example multiplex sensing method for use with the continuous analyte sensors of **FIGs. 3A-3B****,** according to some embodiments disclosed herein. In certain embodiments, signal-processing methods, e.g., filtering, can be utilized to deconvolute different analyte signals collected by the same ammeter (e.g., an ammeter integrated or communicatively coupled with potentiostat) but different working electrodes. In certain embodiments, machine learning models can be utilized for feature extraction to distinguish analyte signals collected by the same ammeter but different working electrodes. For example, such models can be utilized where multiplexing is not available, e.g., where the sensor comprises a potentiostat without a multiplexer interface or the sensor comprises a single working electrode.

In certain embodiments, machine learning models may be particularly beneficial where the presence of an analyte (e.g., a detectable concentration of the analyte) or analyte-related event (e.g., an abnormally high or low concentration of the analyte) of one desired analyte is temporally separated from that of a second analyte, e.g., where one analyte is particularly concentrated (e.g., having high levels) in the morning or afternoon and a second analyte is particularly concentrated at night. In such examples, enzymes specific for the first analyte and enzymes specific for the second analyte may be homogenously deposited on the same working electrode surface (e.g., same active surface). During sensing, current readings from the working electrode may be collected in the time domain, e.g., as a time-domain sensor readout, as shown in the top left of **FIG. 3D****.** Such readouts may then be processed via Fourier transformation by, e.g., calibration engine 154 in **FIG. 1****,** into the frequency domain, as shown in the bottom left of **FIG. 3D****.** In the frequency domain, the signals of each of the different analytes, e.g., the first and second analytes, appear at different frequencies as a result of the temporal separation of the analytes. Accordingly, the different frequency responses of the different analytes may be separated digitally, or by filtering, and then separately deconvoluted by machine learning models to recover the time domain signals for each of the analytes, mutually exclusive with regards to the concentrations of the analytes (shown at right in **FIG. 3D**).

### Example Methods and Systems for Calibrating a Continuous Multi-Analyte Sensor Using Continuously Monitored Analyte Data

### Automatic Correction of Factory Calibration

Systems and methods for in vitro calibration of continuous analyte sensors by the manufacturer (e.g., factory calibration), without reliance on recalibration, may be inadequate with respect to high levels of sensor accuracy required for diabetes management. In some instances, this can be partially attributed to initial factory calibration of continuous analyte sensors being erroneous due to, e.g., manufacturer mischaracterization of the sensitivity of the resistance layer of the sensors. In some instances, this can also be partially attributed to changes in sensor properties (e.g., sensor sensitivity) prior to use by the user. Thus, continuous analyte sensors are typically recalibrated at the beginning of wearing by the user to ensure sensor accuracy.

Conventional in vivo continuous analyte sensing technology has relied on reference measurements performed during a sensor session for recalibration of the continuous analyte sensor. The reference measurements are matched with substantially time corresponding sensor data to create matched pairs. Regression is then performed on the matched data pairs (e.g., by using least squares regression) to generate a conversion function that defines a relationship between a sensor signal and an estimated glucose concentration.

In critical care settings, recalibration of continuous analyte sensors is sometimes performed by using, as reference, a calibration solution with a known concentration of the analyte. This recalibration procedure can be cumbersome, as a calibration bag, separate from an intravenous (IV) bag, is typically used. In an ambulatory setting, recalibration of continuous analyte sensors is sometimes performed by capillary blood measurements (e.g., a finger stick glucose test), through which reference data is obtained and input into the continuous analyte sensor system. This recalibration procedure typically involves frequent finger stick measurements, which can be inconvenient and painful. Thus, conventional methods of recalibration after initial factory calibration involves periodic inputs of reference data, whether they are associated with a calibration solution or with a finger stick measurement, which can be very burdensome to the patient in an ambulatory setting, or to the hospital staff in the critical care setting.

Described herein are systems and methods for automatically recalibrating continuous analyte sensors, such as continuous multi-analyte sensors, using data for at least one analyte in combination with other sensor data, to correct for erroneous factory calibrations or changes in sensor properties. Such systems and methods are particularly beneficial when events affecting calibration sensitivity (or a component causing an error) are shared between multiple analytes on a continuous multi-analyte sensor, multiple analyte readings are abnormal or atypical, and at least one other analyte is exhibiting an expected concentration range during a wear period. The systems and methods are capable of achieving high levels of accuracy, without (or with reduced) reliance on reference data form a reference analyte monitor (e.g., from a blood glucose meter). Therefore, certain systems and methods described herein provide a technical solution to the technical problems described above in the field of sensor calibration.

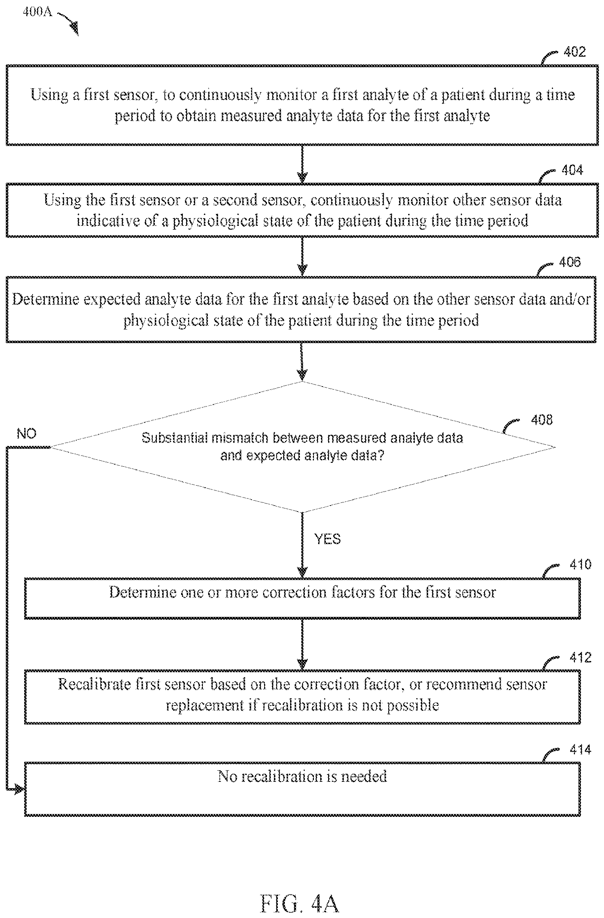

**FIG. 4A** illustrates a flow diagram of an example method 400A for recalibrating a continuous analyte sensor for a first analyte using data for the first analyte in combination with other sensor data to correct for erroneous factory calibration, or changes in sensor properties, in accordance with certain aspects of the present disclosure. For example, method 400A may be performed to recalibrate one or more continuous analyte sensors 202 of continuous analyte monitoring system 104, as illustrated in **FIGs. 1** and **2****.** Thus, method 400A is described below with reference to **FIGs. 1** and **2** and their components.

At block 402, method 400A begins by using a first, factory-calibrated sensor to continuously or non-continuously monitor a first analyte of a patient, such as user 102 illustrated in **FIG. 1****,** during a time period to obtain measured analyte data for the first analyte. Block 402 may be performed by continuous analyte monitoring system 104 illustrated in **FIGs. 1** and **2****,** and more specifically, continuous analyte sensor(s) 202 illustrated in **FIG. 2****,** in certain embodiments. For example, continuous analyte monitoring system 104 may comprise a factory-calibrated continuous glucose sensor 202 to measure the user's glucose levels. Alternatively, continuous analyte monitoring system 104 may comprise a factory-calibrated continuous multi-analyte sensor configured to measure at least the user's glucose levels. Generally, the first analyte may include glucose, though other analytes are also contemplated.

At block 404, method 400A continues by continuously or non-continuously monitoring other sensor data of the user during the time period to obtain measured other sensor data that is indicative of a physiological state of the user. The physiological state of the patient may refer to the general condition or bodily state of the user, including any physical stresses thereon. For example, in certain embodiments, the physiological state of the user includes the engagement of the user in physical activity or rest during the time period, the consumption of a meal by the user during the time period, the administration of a medication to the user during the time period, the occurrence of a glycemic event (e.g., hypo- or hyperglycemia), etc. As another example, the physiological state may include an oxygen level of the user or a temperature of the user. The continuous analyte monitoring system 104 may include an oxygen sensor or a temperature sensor that measures the oxygen level or the temperature of the user. In some instances, the oxygen sensor or the temperature sensor may be combined with or embodied in a continuous analyte sensor 202. As another example, the physiological state may include motion data indicating whether the user is moving or stationary. The continuous analyte monitoring system 104 may include an accelerometer that detects when the user is moving.

The physiological state of the patient may be indicated by a variety of different types of sensor data, either alone or in combination. In certain embodiments, the other sensor data monitored at block 404 includes measured analyte data for one or more additional analytes, including at least a second analyte of the patient, during the time period. For example, in certain embodiments, the other sensor data includes measured analyte data (e.g., concentration values, trends, and/or patterns) for lactate, ketones, glycerol, electrolytes such as sodium and potassium, calculated measurements such as anion gap, and/or any other suitable analytes described herein or combinations thereof. In certain other embodiments, the other sensor data monitored at block 404 includes secondary sensor data, such as one or more of accelerometer data, gyrometer data, acoustic data, global positioning system (GPS) data, heart rate, heart rate reserve, heart rate variability (HRV), electrocardiogram (EKG) data, sweat, electromyogram (EMG), respiration rate, temperature, blood pressure, galvanic skin response, oxygen uptake data (e.g., VO₂ max), sleep, impedance data, etc. In certain embodiments, the other sensor data may be utilized to calculate metrics, such as metrics 130 in **FIG. 1****,** to further inform analysis for recalibrating the first sensor.

In certain embodiments, block 404 is performed by the same sensor as block 402, such as a continuous analyte sensor 202 of continuous analyte monitoring system 104 illustrated in **FIGs. 1** and **2** (e.g., continuous analyte sensor 202 may include a continuous multi-analyte sensor configured to measure both a second analyte and the first analyte). In other embodiments, block 404 is performed a second sensor different than the first sensor used in block 402.

At block 406, calibration engine 154 processes the measured other sensor data and determines expected analyte data for the first analyte based at least partially on the measured other sensor data and/or indicated physiological state of the user during the time period. The expected analyte data for the first analyte refers to predicted analyte data for the first analyte of the patient during the time period, wherein the predictions are informed by the indicated physiological state and/or measured other sensor data, but not the measured analyte data for the first analyte. In certain embodiments, the expected analyte data for the first analyte is determined utilizing predetermined mappings (i.e., correlations) of historical (i.e., previously measured) analyte data for the first analyte to other sensor data and/or physiological states of the user. For example, where the first analyte is glucose and the other sensor data includes lactate data and heart rate data, calibration engine 154 may utilize a historical relationship of glucose, lactate, and heart rate data to determine the expected glucose data based on measured (e.g., current) lactate data and heart rate data. In certain embodiments, the expected analyte data determined at block 406 is a range (e.g., a normal range) for the first analyte based at least partially on the measured other sensor data and/or indicated physiological state of the patient during the time period. For example, where the first analyte is glucose and the other sensor data includes lactate data, accelerometer data, and heart rate data, calibration engine 154 may determine a normal range for glucose based on the lactate data, accelerometer data, and heart rate data.

In certain embodiments, the expected analyte data for the first analyte is determined utilizing one or more trained machine learning models, e.g., trained machine learning models provided by training server system 140 in **FIG. 1****,** or other AI models. In certain embodiments, such models may be configured to use, as input, measured data from one or more sensors measuring multiple analytes, to provide, as output, determinations utilized in recalibration of a continuous analyte sensor. Example inputs for such models may, for example, include continuous analyte sensor data (e.g., for the first analyte and any other analytes), non-analyte sensor data, time, etc. Accordingly, given the interaction of various physiological states, parameters and/or thresholds of such algorithms or models may be altered based, at least in part, on a number of analytes or characteristics being measured for input to reflect the knowledge attained from each of the other analytes or characteristics being measured or the physical states associated with the additional analytes or characteristics being measured.

At block 408, calibration engine 154 compares the expected analyte data for the first analyte (obtained from block 406) and the measured analyte data for first analyte (obtained from block 402) to determine whether there is a substantial mismatch therebetween. A substantial mismatch may be indicative of an error in calibration of one or more of the analyte sensors. Substantial mismatch, in certain embodiments, may refer to a numerical delta (i.e., deviation or dissimilarity) in analyte data that is greater than a predetermined and acceptable range or threshold. For example, a substantial mismatch between the expected analyte data for the first analyte and the measured analyte data for first analyte may occur when the data values diverge (i.e., differ) by more than a predetermined threshold of: 1%, 2%, 3%, 4%, 5%, 6%, 7%, 8%, 9%, 10%, 11%, 12%, 13%, 14%, 15%, 16%, 17%, 18%, 19%, 20%, or more. In certain embodiments, a substantial mismatch between the expected analyte data for the first analyte and the measured analyte data for first analyte includes differing concentration values, trends, patterns, and/or other analyte data dynamics. In embodiments where the expected analyte data determined at block 406 is a range for the first analyte, then "substantial mismatch" at block 408 may refer to the measured analyte data for the first analyte being outside the determined range for the first analyte. For example, for glucose, "substantial mismatch" at block 408 may refer measured glucose data being above or below normal thresholds for glucose of the user.

In certain embodiments, in order to determine whether there is a substantial mismatch between the expected analyte data for the first analyte and the measured analyte data for first analyte, one or more data points of the expected analyte data may be matched with substantially time corresponding data points of the measured analyte data for first analyte to provide one or more matched data pairs. In certain embodiments, one expected analyte data point is matched to one time-corresponding measured analyte data point to form a matched data pair. In other embodiments, a plurality of expected analyte data points are combined (e.g., equally or non-equally weighted average, mean-value, median, or the like) and matched to one time-corresponding measured analyte data point to form a matched data pair. In certain embodiments, one or more matched data pairs may be used in recalibration.

If calibration engine 154 determines that there is no substantial mismatch between the expected analyte data for the first analyte and the measured analyte data for first analyte (i.e., the measured analyte data is "normal"), then no recalibration of the first sensor for the first analyte is needed. In other words, the first sensor is acceptably accurate for the first analyte, or providing measured analyte data within a predetermined and acceptable range of accuracy. Accordingly, method 400A proceeds to block 414, wherein no recalibration is performed.

If calibration engine 154 determines that a substantial mismatch exists between the measured analyte data for the first analyte and the expected analyte data for the first analyte (i.e., the measured analyte data is "abnormal"), then at block 410, calibration engine 154 determines one or more correction factors for measurements of the first analyte taken the first sensor. The one or more correction factors may indicate an error in calibration of the first sensor. In certain embodiments, the one or more correction factors are based at least partially on a degree of mismatch between the measured analyte data for the first analyte and the expected analyte data for the first analyte. In certain embodiments, the one or more correction factors are based at least partially on the predetermined mappings of historical analyte data for the first analyte to the other sensor data and/or physiological states of the patient. In certain embodiments, the one or more correction factors may generally include an adjustment to a sensitivity (m), rate of change of sensitivity (ddm/ddt), baseline/intercept (b), rate of change of baseline (ddb/ddt), sensitivity profile, relationships between particular stimulus signal outputs (e.g., impedance) to sensor sensitivity, relationships between particular stimulus signal outputs to sensor temperature or temperature, and/or calibration code associated with a sensor being recalibrated, for the first sensor. In certain embodiments, a plurality of possible values for each of the one or more correction factors may be determined, to which a weighting or averaging function may be applied. In certain embodiments, new values may be determined for one or more sensor calibration parameters (e.g., sensitivity (m), baseline/intercept (b), etc.) without an adjustment of a prior known calibration parameter via one or more correction factors when there is a determination that substantial mismatch exists between the measured analyte data for the first analyte and the expected analyte data for the first analyte.

At block 412, the first sensor may be automatically recalibrated for the first analyte based at least partially on the determined one or more correction factors. In other words, the calibration engine 154 may be configured to convert at least one sensor data point received from the first sensor for the first analyte into recalibrated data, in real time, based at least in part on the correction factors and/or the sensor calibration parameters (e.g., sensitivity and/or baseline) determined at block 410. In certain embodiments, recalibration of the first sensor is performed after a determination by the calibration engine 154 that recalibration of the first sensor is possible. If, however, calibration engine 154 determines that recalibration of the first sensor is not possible, then, in certain embodiments, the data stream from the first sensor may be terminated, and a notification or alert may be generated and provided to the patient, e.g., via display 107 in **FIG. 1****,** recommending replacement of the first sensor.

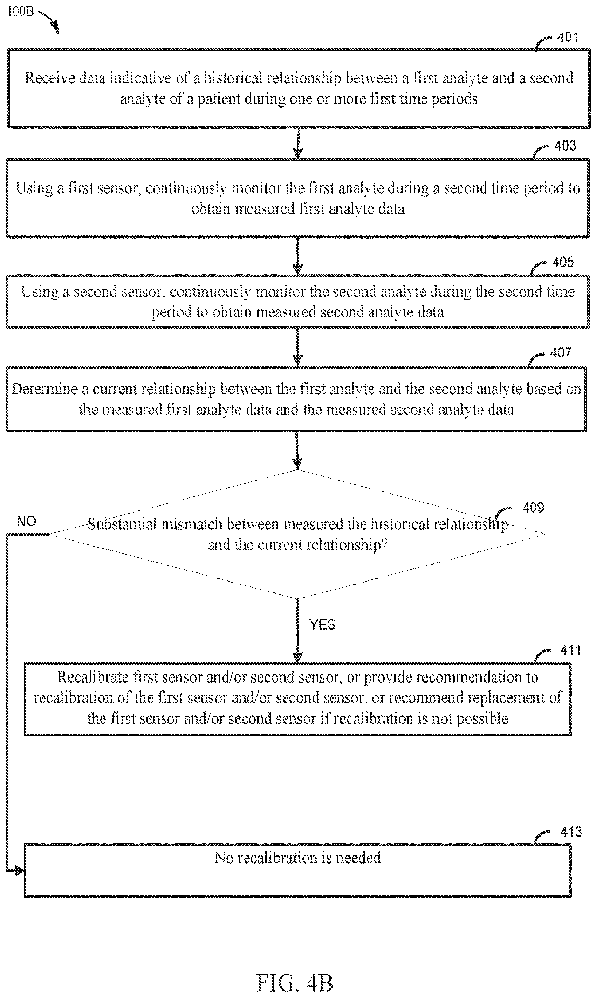

**FIG. 4B** illustrates a flow diagram of another example method 400B for recalibrating one or more continuous analyte sensors using data for a first analyte and a second analyte to correct for erroneous factory calibration, or changes in sensor properties, in accordance with certain aspects of the present disclosure. In the example of FIG. 4B, one or more sensors for a first analyte and/or a second analyte may be recalibrated. Similar to method 400A, method 400B may be performed to recalibrate one or more continuous analyte sensors 202 of continuous analyte monitoring system 104, as illustrated in **FIGs. 1** and **2****.** Thus, method 400B is described below with reference to **FIGs. 1** and **2** and their components.

At block 401, method 400B begins with the calibration engine receiving (or accessing) data indicative of a historical relationship between a first analyte and a second analyte of a patient, such as user 102 illustrated in **FIG. 1****,** during one or more first time periods. In certain embodiments, the data, which may be raw or processed data, may be received from, e.g., user database 110 and accessed by, e.g., DAM 155. "Historical relationship" as used herein may refer to historical mappings (i.e., correlations) between historical (i.e., previously measured) analyte data for the first analyte and historical analyte data for the second analyte. Such historical mappings may be based at least partially on one or more matched data pairs comprising one or more data points of historical analyte data for the first analyte matched with substantially time corresponding data points of historical analyte data for the second analyte during the first time period. In certain embodiments, one historical analyte data point for the first analyte is matched to one time-corresponding historical analyte data point for the second analyte to form a matched data pair. In other embodiments, a plurality of historical analyte data points for the first analyte are combined (e.g., equally or non-equally weighted average, mean-value, median, or the like) and matched to one time-corresponding historical analyte data point for the second analyte, or vice versa, to form a matched data pair. In certain embodiments, one or more matched data pairs during the first time period facilitate determination of the historical relationship.

In certain embodiments, the first analyte and/or the second analyte include glucose, lactate, ketones, glycerol, electrolytes such as sodium and potassium, calculated measurements such as anion gap, or any other suitable analytes described herein or combinations thereof. In certain embodiments, the first analyte comprises glucose, and the second analyte comprises one or more of lactate, ketones, glycerol, electrolytes such as sodium and potassium, calculated measurements such as anion gap, or any other suitable analytes described herein. In specific embodiments, the first analyte comprises glucose, and the second analyte comprises lactate.

At block 403, using a first, factory-calibrated sensor, the first analyte of the patient is monitored continuously or non-continuously during a second time period to obtain measured analyte data for the first analyte. Block 403 may be performed by continuous analyte monitoring system 104 illustrated in **FIGs. 1** and **2****,** and more specifically, continuous analyte sensor(s) 202 illustrated in **FIG. 2****,** in certain embodiments. For example, continuous analyte monitoring system 104 may comprise a factory-calibrated continuous glucose sensor 202 to measure the user's glucose levels. Alternatively, continuous analyte monitoring system 104 may comprise a factory-calibrated continuous multi-analyte sensor configured to measure at least the user's glucose levels. As described above, the first analyte may include glucose, though other analytes are also contemplated. In still other embodiments, a fingerstick sample may be taken and utilized to obtain measured analyte data for the first analyte.

At block 405, method 400B continues by continuously or non-continuously monitoring the second analyte of the patient during the second time period to obtain measured analyte data for the second analyte. In certain embodiments, block 405 is performed by the same sensor as block 403, such as a continuous analyte sensor 202 of continuous analyte monitoring system 104 illustrated in **FIGs. 1** and **2** (e.g., continuous analyte sensor 202 may include a continuous multi-analyte sensor configured to measure both the second analyte and the first analyte). In other embodiments, block 405 is performed a second factory-calibrated sensor different than the first sensor used in block 403. In still other embodiments, a fingerstick sample may be taken and utilized to obtain measured analyte data for the second analyte.

At block 407, calibration engine 154 processes the measured first analyte data and the measured second analyte data and determines a current relationship between the first analyte and the second analyte during the second time period (i.e., determines the current relationship based on the measured first analyte data and the measured second analyte data). For example, the analyte measurements taken during the second time period are processed to determine directional trends, which may include data such as a minimum measurement of an analyte during the second time period, a maximum measurement of an analyte during the second time period, a direction of change (e.g., upward rise or downward fall) of analyte levels during the second time period, a rate of change of analyte levels during the second time period, etc. For example, for lactate, lactate trends may include lactate production rates, lactate clearance rates, lactate threshold, etc., during the second time period.

The current relationship may comprise mappings (i.e., correlations) between measured analyte data for the first analyte and measured analyte data for the second analyte during the second time period. Such mappings may be based at least partially on one or more matched data pairs comprising one or more data points of measured analyte data for the first analyte matched with substantially time corresponding data points of measured analyte data for the second analyte during the second time period. In certain embodiments, one measured analyte data point for the first analyte is matched to one time-corresponding measured analyte data point for the second analyte to form a matched data pair. In other embodiments, a plurality of measured analyte data points for the first analyte are combined (e.g., equally or non-equally weighted average, mean-value, median, or the like) and matched to one time-corresponding measured analyte data point for the second analyte, or vice versa, to form a matched data pair. In certain embodiments, one or more matched data pairs during the first time period facilitate determination of the current relationship.

In certain embodiments, block 407 further comprises processing the received data (e.g., received raw data) indicative of the historical relationship to determine the historical relationship in a manner substantially similar to determining the current relationship, as described above. Note that in some embodiments, however, the data indicative of the historical relationship is already processed prior to or upon receipt at block 401, and therefore, the historical relationship is already determined prior to determination of the current relationship at block 405. In certain embodiments, the current and/or historical relationship is determined utilizing one or more trained machine learning models, e.g., trained machine learning models provided by training server system 140 in **FIG. 1****,** or other AI models. In certain embodiments, such models may be configured to use, as input, measured data from one or more sensors measuring multiple analytes to provide, as output, determinations utilized in recalibration of a continuous analyte sensor. Example inputs for such models may, for example, include continuous analyte sensor data (e.g., for the first analyte and any other analytes), non-analyte sensor data, time, etc. Accordingly, given the interaction of various physiological states, parameters and/or thresholds of such algorithms or models may be altered based, at least in part, on a number of analytes or characteristics being measured for input to reflect the knowledge attained from each of the other analytes or characteristics being measured or the physical states associated with the additional analytes or characteristics being measured.

At block 409, calibration engine 154 compares the current relationship between the first analyte and the second analyte and the historical relationship between the first analyte and the second analyte to determine whether there is a substantial mismatch therebetween. Substantial mismatch, in certain embodiments, may refer to a numerical delta in data that is greater than a predetermined and acceptable range or threshold. For example, a substantial mismatch between the current relationship and the historical relationship may occur when the data values diverge (i.e., differ) by more than a predetermined threshold of: 1%, 2%, 3%, 4%, 5%, 6%, 7%, 8%, 9%, 10%, 11%, 12%, 13%, 14%, 15%, 16%, 17%, 18%, 19%, 20%, or more. In certain embodiments, a substantial mismatch between the current relationship and the historical relationship includes differing concentration values, trends, patterns, and/or other analyte data dynamics.

If calibration engine 154 determines that there is no substantial mismatch between the current relationship and the historical relationship (i.e., the current relationship is "normal"), then no recalibration of the first sensor for the first analyte or second analyte is needed. In other words, the first sensor is acceptably accurate for the first analyte and the second analyte, or providing measured analyte data within a predetermined and acceptable range of accuracy. Accordingly, method 400B proceeds to block 413, wherein no recalibration is performed.

If calibration engine 154 determines that a substantial mismatch exists between the current relationship and the historical relationship (i.e., the current relationship is "abnormal"), then at block 411, calibration engine 154 either generates and provides a notification or alert to the patient, e.g., via display 107 in **FIG. 1****,** recommending recalibration of the one or more sensors for measurements of the first analyte and/or the second analyte, or, calibration engine 154 automatically recalibrates one or more sensors for the first analyte and/or second analyte based at least partially on the historical relationship. In other words, the calibration engine 154 may be configured to convert at least one sensor data point received from one or more sensors for the first and/or second analytes into recalibrated data, in real time, based at least in part on the historical relationship.

In certain embodiments, determining which sensor(s) for the first analyte and/or the second analyte require recalibration is further based on mappings or correlations between the measured first analyte data, the measured second analyte data, and/or secondary sensor data. Examples of secondary sensor data include accelerometer data, gyrometer data, global positioning system (GPS) data, heart rate, heart rate reserve, heart rate variability (HRV), electrocardiogram (EKG) data, electromyogram (EMG), respiration rate, temperature, blood pressure, galvanic skin response, oxygen uptake data (e.g., VO₂ max), sleep, impedance data, strain sensor data, etc. In certain embodiments, determining which sensor(s) for the first analyte and/or the second analyte require recalibration is further based on mappings or correlations between the measured first analyte data, the measured second analyte data, and/or measured analyte data for one or more additional analytes. For example, where the first analyte is glucose and the second analyte is lactate, the one or more additional analytes may include ketones, glycerol, potassium (e.g., hyperkalemia), sodium, CO₂ or anion-gap, or similar analytes.

In certain embodiments, determining which sensor(s) for the first analyte and/or the second analyte require recalibration is further based on expected analyte data for the first analyte and/or expected analyte data for the second analyte. Such expected analyte data may be based at least partially on other sensor data, e.g., measured secondary sensor data and/or measured analyte data for the one or more additional analytes. The expected analyte data for the first and/or second analytes refers to predicted analyte data for the first and/or second analytes of the patient during the second time period, wherein the predictions are informed by e.g., measured secondary sensor data and/or measured analyte data for the one or more additional analytes, but not the measured analyte data for the first or second analytes. In certain embodiments, the expected analyte data for the first and/or second analytes is determined utilizing predetermined mappings (e.g., correlations) of historical (e.g., previously measured) analyte data for the first and/or second analytes to other sensor data. In certain embodiments, the expected analyte data for the first and/or second analytes is a range (e.g., a normal range) for the first and/or second analytes, based at least partially on other sensor data of the patient during the second time period. For example, where the first analyte is glucose and the second analyte is lactate, the other sensor data may include, e.g., ketone levels, accelerometer data, and heart rate data.

In certain embodiments, prior to providing the recommendation for recalibration or automatically recalibrating one or more sensors, the calibration engine 154 first determines whether recalibration of the one or more sensors for measurements of the first analyte and/or the second analyte is possible. If calibration engine 154 determines that recalibration is not possible, then instead of providing a recommendation of recalibration or recalibrating the sensor(s), in certain embodiments, the data stream from the one or more sensors for the first analyte and/or the second analyte may be terminated, and a notification or alert may be generated and provided to the patient recommending replacement of the one or more sensors.

### Determining Optimal Calibration Time

As described above, conventional in vivo continuous analyte sensing technology has typically relied on reference measurements performed during a sensor session for recalibration of a continuous analyte sensor in order to account for changes in sensor sensitivity and/or stability over time and ensure accurate measuring by the sensor. In critical care settings, recalibration of continuous analyte sensors is sometimes performed by using a calibration solution with a known concentration of the analyte as a reference. In ambulatory settings, recalibration of continuous analyte sensors is sometimes performed by finger stick sample measurements, through which reference data is obtained and input into the continuous analyte sensor system. Though not required by some recent continuous analyte sensing systems, obtaining such reference measurements for recalibration is useful in minimizing any bias introduced by imperfections in factory calibration algorithms and/or sensor manufacturing processes, as well as for measuring alternative analytes and for special applications of continuous analyte sensing systems, such as, e.g., in-hospital and/or post-discharge monitoring, monitoring patients with diminished cerebral glucose metabolism (dCGM), etc.

Typically, reference measurements are collected and continuous analyte sensors are recalibrated at the beginning or middle of wearing periods by the user. It is preferable to collect such reference samples for recalibration when the patient's analyte levels are stable, and thus, are within a normal range for the patient, or during another period of interest wherein analyte levels are exhibiting expected, or normal, behavior. For example, with glucose, the patient's levels thereof typically change most prominently right after consumption of a meal. Because it takes time to reach equilibrium between blood glucose levels and interstitial fluid glucose levels, there is a lag between e.g., a finger stick sample values (e.g., blood) and measured sensor values (e.g., interstitial fluid). Thus, it is preferable not to recalibrate a continuous analyte sensor immediately following a meal. A similar preference applies for recalibration post-exercise.

However, patients do not always abide by recommendations or guidelines regarding when to recalibrate their continuous analyte sensors. For example, many patients obtain their continuous analyte sensors from in-store pharmacies or similar channels, which may not always provide the patients with complete recalibration instructions (e.g., when is the best time to recalibrate). Thus, some patients may not be made aware of optimal times for recalibrating their continuous analyte sensors. Moreover, patients may sometimes believe they are recalibrating during an optimal time period, but their levels may, in fact, be unstable during a particular time.

Described herein are systems and methods for automatically determining optimal calibration times for continuous analyte sensors, such as continuous multi-analyte sensors, using measured analyte data for at least one analyte, optionally in combination with other sensor data. Such systems and methods may notify a patient of the determined optimal calibration times, thus facilitating user-directed recalibration during periods when the patient's analyte levels are stable, without relying on any timing guesswork by the patient. Such systems and methods may also automatically calibrate an analyte sensor during an optimal calibration time, once the optimal calibration time is automatically determined using the techniques described herein.

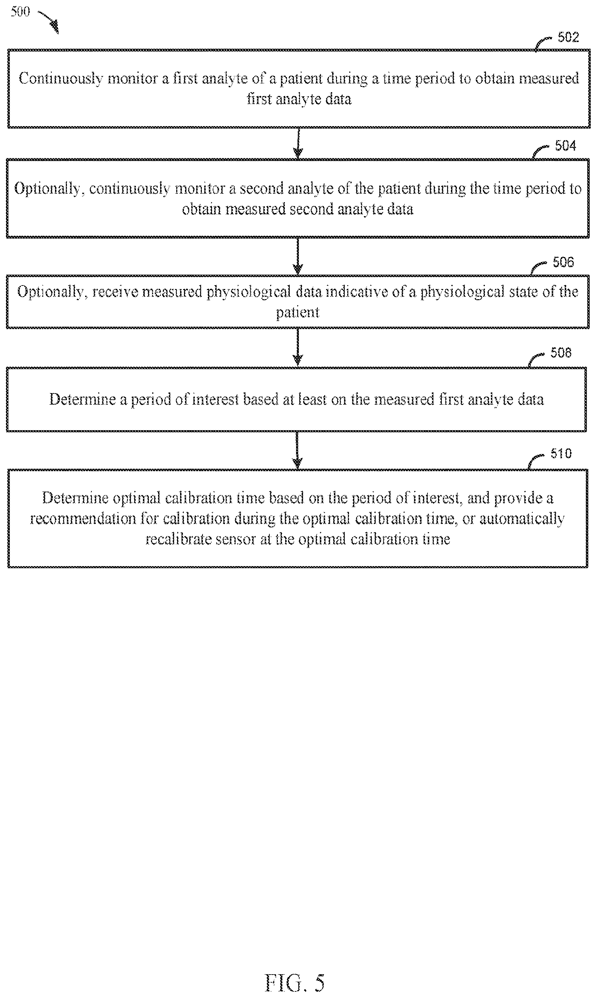

**FIG. 5** illustrates a flow diagram of an example method 500 for determining an optimal calibration time for a continuous analyte sensor measuring a first analyte using measured analyte data for at least the first analyte, optionally in combination with other sensor data, in accordance with certain aspects of the present disclosure. Method 500 may be performed to determine optimal calibration times for one or more continuous analyte sensors 202 of continuous analyte monitoring system 104, as illustrated in **FIGs. 1** and **2****.** Thus, method 500 is described below with reference to **FIGs. 1** and **2** and their components.

At block 502, method 500 begins by using a first, factory-calibrated sensor to continuously or non-continuously monitor a first analyte of a patient, such as user 102 illustrated in **FIG. 1****,** during a time period to obtain measured analyte data for the first analyte. Block 502 may be performed by continuous analyte monitoring system 104 illustrated in **FIGs. 1** and **2****,** and more specifically, continuous analyte sensor(s) 202 illustrated in **FIG. 2****,** in certain embodiments. For example, continuous analyte monitoring system 104 may comprise a factory-calibrated continuous glucose sensor 202 to measure the user's glucose levels. Alternatively, continuous analyte monitoring system 104 may comprise a factory-calibrated continuous multi-analyte sensor configured to measure at least the user's glucose levels. Generally, the first analyte may include glucose, though other analytes such as lactate, ketones, glycerol, electrolytes such as sodium and potassium, calculated measurements such as anion gap, or any other suitable analytes described herein, are also contemplated.

At block 504, method 500 continues by optionally continuously or non-continuously monitoring one or more additional analytes, including at least a second analyte of the patient, during the time period to obtain measured analyte data for the one or more additional analytes. In certain embodiments, block 504 is performed by the same sensor as block 502, such as a continuous analyte sensor 202 of continuous analyte monitoring system 104 illustrated in **FIGs. 1** and **2** (e.g., continuous analyte sensor 202 may include a continuous multi-analyte sensor configured to measure both a second analyte and the first analyte). In other embodiments, block 504 is performed by a second factory-calibrated sensor different than the first sensor used in block 502.

In certain embodiments, the one or more additional analytes include lactate, ketones, glycerol, electrolytes such as sodium and potassium, calculated measurements such as anion gap, or any other suitable analytes described herein or combinations thereof. In certain embodiments, the first analyte comprises glucose, and a second analyte comprises one or more of lactate, ketones, glycerol, electrolytes such as sodium and potassium, calculated measurements such as anion gap, or any other suitable analytes described herein. In certain embodiments, the first analyte comprises glucose, and a second analyte comprises lactate or ketones. In certain embodiments, the first analyte comprises glucose, a second analyte comprises lactate, and a third analyte comprises ketones.

At block 506, method 500 continues by optionally continuously or non-continuously monitoring other sensor data of the patient during the time period to obtain measured other sensor data that is indicative of a physiological state of the patient. As previously described, the physiological state of the patient may refer to the general condition or bodily state of the user, including any physical stresses thereon. For example, in certain embodiments, the physiological state of the user includes the engagement of the user in physical activity or rest during the time period, the consumption of a meal by the user during the time period, the administration of a medication to the user during the time period, the occurrence of a glycemic event (e.g., hypo- or hyperglycemia), etc.

The physiological state of the patient may be indicated by a variety of different types of sensor data, either alone or in combination. In certain embodiments, the other sensor data optionally monitored at block 506 includes secondary sensor data, such as one or more of accelerometer data, gyrometer data, global positioning system (GPS) data, heart rate, heart rate reserve, heart rate variability (HRV), electrocardiogram (EKG) data, electromyogram (EMG), respiration rate, temperature, blood pressure, galvanic skin response, sweat, oxygen uptake data (e.g., VO₂ max), sleep, impedance data, etc. In certain embodiments, the other sensor data may be utilized to calculate metrics, such as metrics 130 in **FIG. 1****,** to further inform analysis for determining optimal calibration time.

In certain embodiments, block 506 is performed by the same sensor as block 502 and/or block 504, such as a continuous analyte sensor 202 of continuous analyte monitoring system 104 illustrated in **FIGs. 1** and **2** (e.g., continuous analyte sensor 202 may include a continuous multi-analyte sensor configured to measure the first analyte, a second analyte, and/or other sensor data). In other embodiments, block 506 is performed by a third sensor different than the sensor used in block 502 and/or block 504.

At block 508, calibration engine 154 determines a period of interest for the first analyte based on at least the measured first analyte data. The period of interest for the first analyte may be determined by monitoring the first analyte during the time period and identifying a period of expected or normal behavior, which may be at least partially based on one or more concentration thresholds and/or time thresholds for the first analyte. Identifying the period of expected or normal behavior may be based on a concentration of the first analyte, changes in the concentration of the first analyte, the rate of change in the concentration of the first analyte, etc. In certain embodiments, the period of interest is a period of stability for the first analyte, which may be determined by identifying a most stable period during the time period. For example, in certain embodiments, the period of stability for the first analyte is determined by identifying a time period satisfying a minimum time threshold (e.g., as long or longer than a minimum amount of time), wherein the measured analyte is within a minimum and a maximum concentration level corresponding to normal first analyte levels for the user. In certain other embodiments, the period of interest is a period of expected, or normal, patterns or trends for the first analyte, such as a period of expected change for the first analyte. For example, in certain embodiments, the period of interest is a period wherein the measured analyte levels, or a rate of change thereof, for the first analyte are within a predetermined and acceptable range or threshold.

In certain embodiments, the minimum time threshold for the first analyte may be about 1 minute, about 2 minutes, about 3 minutes, about 4 minutes, about 5 minutes, about 6 minutes, about 7 minutes, about 8 minutes, about 9 minutes, about 10 minutes, about 11 minutes, about 12 minutes, about 13 minutes, about 14 minutes, about 15 minutes, about 16 minutes, about 17 minutes, about 18 minutes, about 19 minutes, about 20 minutes, about 21 minutes, about 22 minutes, about 23 minutes, about 24 minutes, about 25 minutes, about 26 minutes, about 27 minutes, about 28 minutes, about 29 minutes, about 30 minutes, or more in duration.

In certain embodiments, the period of interest for the first analyte is further determined by cross-checking, or cross-tabulating, the measured analyte levels for the first analyte with measured analyte levels for the one or more additional analytes including at least the second analyte, and/or measured other sensor data. For example, in certain embodiments, at block 508, calibration engine 154 may determine a period of interest for the first analyte that corresponds with a period of interest for a second analyte, based on at least the measured first analyte data and the measured second analyte data. In further embodiments, at block 508, calibration engine 154 may determine a period of interest for the first analyte that corresponds with a period of interest for the second analyte and a period of interest for the other sensor data, based on the measured first analyte data, the measured second analyte data, and the measured other sensor data. In such examples, the determination of the periods of interest for the second analyte and/or the other sensor data may also be based on the satisfaction of one or more thresholds, such as minimum and maximum value thresholds and minimum time thresholds. In certain examples, the minimum time threshold(s) for the second analyte and/or the other sensor data are the same as the minimum time threshold(s) for the first analyte. In certain other examples, the minimum time threshold(s) for the second analyte and/or the other sensor data are different than the minimum time threshold(s) for the first analyte.

In one specific example, a period of interest for glucose is a period of stability that is determined when: glucose signals (e.g., measured glucose data) from a continuous analyte sensor or continuous multi-analyte sensor are stable, or within a normal range for the patient, for at least 5, 10, 15, 20, 25, or 30 or more minutes; there are no elevated lactate levels for at least 60 minutes, 70 minutes, 80 minutes, or 90 minutes or more (e.g., thus no meal or exercise); and, there is no high step count, high accelerometer data, or high heart rate data measured for 30 minutes, 45, minutes, or 60 or more minutes (e.g., thus no exercise or stress).

Additional parameters that may be utilized at block 508 to determine a period of interest for the first analyte include but are not limited to time of insertion of the continuous analyte sensor or continuous multi-analyte sensor, time of day, sensor signal quality, and the like. A sensor calibration may be more beneficial at the beginning of a period of wearing by the patient, as opposed to later in the period of wearing, for example, to help ensure accuracy of a sensor over its use period. Further, because of the declining chemical stability of a given sensor throughout its lifetime, sensor readings may be prone to error or noise towards the end of a sensor's life; accordingly, more calibrations may be recommended towards the end of the sensor's life. Still further, due to the circadian rhythm of the patient, recalibration may be more beneficial at particular times of a given day (e.g., during times of the day when sensor readings may be more stable).

In certain embodiments, the calibration engine utilizes one or more trained machine learning models, e.g., trained machine learning models provided by training server system 140 in **FIG. 1****,** or other AI models, to predict or determine data associated with a patient's typical daily schedule (e.g., periods of rest versus exercise, periods of meal consumption versus periods of fasting, etc.). Such data associated with the patient's daily schedule may also be utilized as a parameter in determining a period of interest for the first analyte, in addition to or alternatively to the one or more additional analytes or the other sensor data.

In certain embodiments, the period of interest corresponds with an actual time the continuous analyte sensor or continuous multi-analyte sensor takes a measurement of the first analyte, the one or more additional analytes, and/or the other sensor data (e.g., during a data collection interval). In further embodiments, in addition to or alternatively to the data collection interval, the period of interest corresponds with a time that the measurement(s) are displayed to the patient (e.g., during a time display interval). Such data collection and/or time display intervals may occur every 30 seconds, 1 minute, 2 minutes, 3 minutes, 4 minutes, 5 minutes, 6 minutes, 7 minutes, 8 minutes, 9 minutes, 10 minutes, 15 minutes, 20 minutes, 25 minutes, 30 minutes, etc.

After determining a period of interest for at least the first analyte, then at block 510, calibration engine 154 determines an optimal calibration time based on the period of interest and may, in certain embodiments, generate and provide a notification or alert to the patient, e.g., via display 107 in FIG. 1, recommending recalibration of the continuous analyte sensor or continuous multi-analyte sensor (for measurement of the first analyte) during the optimal calibration time. In certain embodiments, the notification or alert is provided to the patient via cell phone or receiver notifications, email, text message, etc. In certain embodiments, the notification or alert is provided in passive form, in which case the patient may need to check their phone (e.g., an application on their phone) or receiver for notifications. In certain embodiments, at block 510, calibration engine 154 automatically calibrates the analyte sensor for the first analyte during the determined optical calibration time based on at least the period of interest, in addition or as an alternative to providing the recommendation to the patient. In certain embodiments, after determining a period of interest for at least the first analyte, the system may be configured to automatically recalibrate by requesting data from a connected reference source (e.g., a system and/or device that obtains and/or analyses a blood-glucose measurement without user intervention). The system may communicate a message to the user or patient indicating that recalibration is needed or requesting that the user or patient perform the recalibration.

In certain embodiments, prior to providing a recommendation for recalibration, the calibration engine 154 first determines whether recalibration of the one or more sensors for measurements of the first analyte and/or the second analyte is possible. If calibration engine 154 determines that recalibration is not possible, then instead of providing a recommendation of recalibration, in certain embodiments, the data stream from the one or more sensors for the first analyte and/or the second analyte may be terminated, and a notification or alert may be generated and provided to the patient recommending replacement of the one or more sensors.

### Artifact Detection and Compensation

### Compression Artifacts

Traditional in vivo continuous analyte sensors, and particularly, continuous glucose sensors and continuous lactate sensors, are regularly affected by compression artifacts, or changes in measured analyte concentration levels caused by the appendage or body part having the sensor worn thereon being compressed. For example, compression artifacts are most commonly observed at nighttime when a patient is sleeping, as the patient may lay on their abdomen, arm, or leg (where a continuous analyte sensor is typically worn), thereby creating external pressure on or around a worn sensor. Compression artifacts can manifest in many different ways in measured analyte data; in many cases, such artifacts show up as an abrupt deviation and recovery of a raw sensor signal.

It is suspected that at least glucose and lactate compression artifacts are caused by the decrease of local blood supply as sensor-adjacent tissues are compressed. As a result of this compression, the cells near the sensor-tissue interface do not receive enough oxygen for aerobic respiration, and thus switch to anaerobic respiration to support cell metabolism, in which glucose is converted to lactate instead of CO₂, making lactate a good indicator of compression events. And, again, due to the decrease of local blood supply, glucose is not resupplied to the compressed tissues fast enough, causing a detected drop in glucose and simultaneous spike in lactate.

Compression artifacts can cause many problems for continuous analytes monitoring systems. For example, compression artifacts may result in burdensome and false alerts, gaps in measured analyte data, unnecessary or erroneous recommendations for treatments, erroneous decisions by automated insulin delivery (AID) systems, false adjustment of manual insulin delivery, inaccurate changes in retrospective glycemic metrics, and/or inaccurate analyte trend or pattern analysis. Thus, there is a need for systems and methods for automatically detecting a compression event of a patient, thus enabling appropriate action to be thereafter taken to address the compression event and avoid any errors in measured analyte data analysis and/or treatment of the patient. Described herein are systems and methods for automatically detecting compression events.

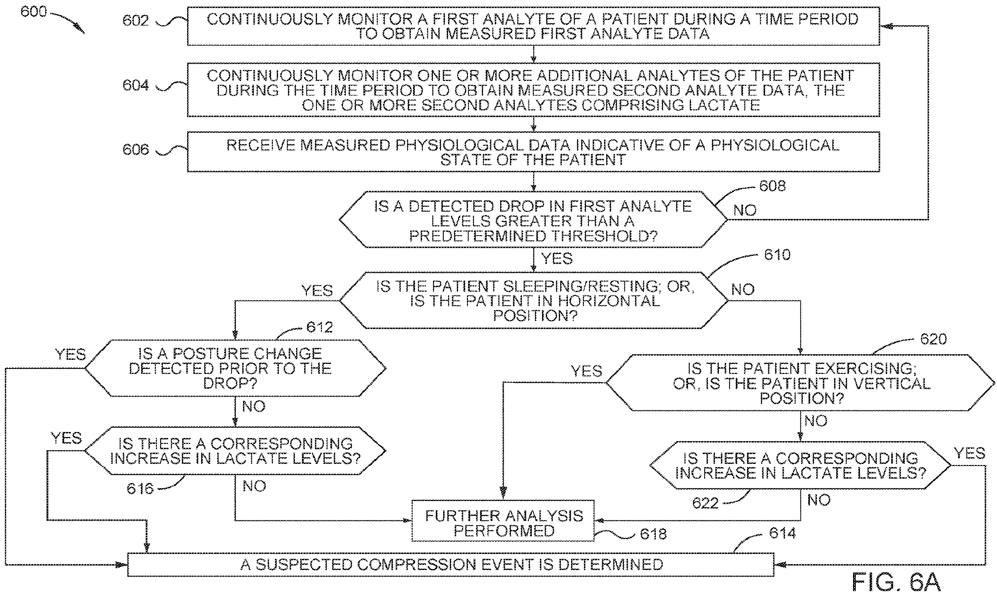

**FIG. 6A** illustrates a flow diagram of an example method 600 for automatically detecting the occurrence of a compression artifact, in accordance with certain aspects of the present disclosure. Method 600 may be performed using one or more continuous analyte sensors 202 of continuous analyte monitoring system 104, as illustrated in **FIGs. 1** and **2****.** Thus, method 600 is described below with reference to **FIGs. 1** and **2** and their components.

At block 602, method 600 begins by continuously or non-continuously monitoring a first analyte of a patient, such as user 102 illustrated in **FIG. 1****,** during a time period to obtain measured analyte data for the first analyte. Block 602 may be performed by continuous analyte monitoring system 104 illustrated in **FIGs. 1** and **2****,** and more specifically, continuous analyte sensor(s) 202 illustrated in **FIG. 2****,** in certain embodiments. For example, continuous analyte monitoring system 104 may comprise a continuous glucose sensor 202 to measure the user's glucose levels. Alternatively, continuous analyte monitoring system 104 may comprise a continuous multi-analyte sensor configured to measure at least the user's glucose levels. Generally, the first analyte may include glucose, though other analytes such as ketones, glycerol, electrolytes such as sodium and potassium, calculated measurements such as anion gap, or any other suitable analytes described herein, are also contemplated.

At block 604, method 600 continues by continuously or non-continuously monitoring one or more additional analytes comprising at least lactate during the time period to obtain measured analyte data for the one or more additional analytes. In certain embodiments, block 604 is performed by the same sensor as block 602, such as a continuous analyte sensor 202 of continuous analyte monitoring system 104 illustrated in **FIGs. 1** and **2** (e.g., continuous analyte sensor 202 may include a continuous multi-analyte sensor configured to measure both glucose and lactate). In other embodiments, block 604 is performed a second sensor different from the first sensor used in block 602.

As described above, the one or more additional analytes include at least lactate, which may be a good indicator of compression events, particularly when utilized in combination with measured glucose data. In certain embodiments, the one or more additional analytes further include one or more of ketones, glycerol, electrolytes such as sodium and potassium, calculated measurements such as anion gap, or any other suitable analytes described herein or combinations thereof that may provide an indicia of the occurrence of a compression event.

At block 606, method 600 continues by receiving measured physiological data indicative of a physiological condition of the patient during the time period. The physiological state of the patient, as described above, may refer to the general condition or bodily state of the user, including any physical stresses thereon. For example, in certain embodiments, the physiological state of the user includes the engagement of the user in physical activity or rest during the time period, the consumption of a meal by the user during the time period, the administration of a medication to the user during the time period, the occurrence of a glycemic event (e.g., hypo- or hyperglycemia), etc.

The measured physiological data at block 606 may include other sensor data measured by one or more other sensors during the time period. In certain embodiments, the measured physiological data received at block 606 includes measured analyte data for one or more additional analytes during the time period. For example, in certain embodiments, the measured physiological data includes measured analyte data (e.g., concentration values, trends, and/or patterns) for ketones, glycerol, electrolytes such as sodium and potassium, calculated measurements such as anion gap, and/or any other suitable analytes described herein or combinations thereof.

In certain embodiments, the measured physiological data received at block 606 includes secondary sensor data measured during the time period, such as one or more of accelerometer data, gyrometer data, global positioning system (GPS) data, heart rate, heart rate reserve, heart rate variability (HRV), electrocardiogram (EKG) data, electromyogram (EMG), respiration rate, temperature, blood pressure, galvanic skin response, oxygen uptake data (e.g., VO₂ max), sleep, impedance data, etc. In certain embodiments, the measured physiological data may be utilized to calculate metrics, such as metrics 130 in **FIG. 1****,** to further inform the determination of a compression event.

In certain embodiments, the measured physiological data at block 606 is received from the same sensor utilized in block 602 and/or 604, such as a continuous analyte sensor 202 of continuous analyte monitoring system 104 illustrated in **FIGs. 1** and **2** (e.g., continuous analyte sensor 202 may include a continuous multi-analyte sensor configured to measure multiple analytes). In other embodiments, the measured physiological data at block 606 is received from a sensor different than the sensor used in block 602 and/or 604.

At block 608, method 600 continues with calibration engine 154 processing the measured first analyte data to detect the occurrence of a change, such as a drop (e.g., decrease or downward fall) in first analyte levels that satisfies one or more predetermined thresholds. For example, the first analyte measurements taken during the time period are processed to determine directional trends, which may include data such as a minimum measurement of the first analyte during the time period, a maximum measurement of the first analyte during the time period, a direction of change (e.g., upward rise or downward fall) of first analyte levels during the time period, a rate of change of first analyte levels during the time period, etc.

In certain embodiments, the one or more predetermined thresholds include concentration thresholds. For example, in certain embodiments, the one or more predetermined thresholds include a minimum first analyte concentration threshold, which may be satisfied upon the patient's measured first analyte values substantially equaling, hovering around, or falling below the minimum first analyte concentration threshold. In certain embodiments, the one or more predetermined thresholds include rate of change thresholds. For example, in certain embodiments, the one or more predetermined thresholds include a maximum first analyte rate of change threshold, which may be satisfied upon the patient's measured first analyte values declining at a rate substantially equal to or greater than the maximum first analyte rate of change threshold. In certain embodiments, the one or more predetermined thresholds include time period thresholds, which may be utilized in combination with one or more concentration thresholds and/or rate of change thresholds. For example, in certain embodiments, the one or more predetermined thresholds include a maximum time period threshold, which may be satisfied upon the patient's measured first analyte values substantially equaling, hovering around, or falling below the minimum first analyte concentration threshold, and/or the patient's measured first analyte values declining at a rate substantially equal to or greater than the maximum first analyte rate of change threshold, for a time period substantially equal to or greater than the maximum time period threshold.

If the detected drop in first analyte levels does not satisfy the one or more predetermined thresholds, then the continuous monitoring of the first analyte at block 602 continues. If, however, the detected drop in first analyte levels does satisfy the one or more predetermined thresholds, then method 600 proceeds to block 610.

In certain embodiments, at block 610, calibration engine 154 determines whether the patient (e.g., user 102) is sleeping or resting. Sleeping, or resting, may be a good indicator that a drop in first analyte levels is caused by a compression event, as compression events are most commonly observed when a patient is sleeping and lying on the body part on which their continuous analyte sensor is being worn. In such embodiments, in order to determine whether the patient is sleeping, calibration engine 154 may process and correlate measured analyte data for the first analyte and/or one or more additional analytes from blocks 602 and 604, in addition to one or more types of the received physiological data from block 606. In specific examples, calibration engine 154 may determine whether the patient is sleeping by processing and mapping (e.g., correlating) measured analyte levels for, e.g., glucose and lactate, in addition to heart rate data, accelerometer data, respiration data, blood pressure data, and/or other suitable types of secondary sensor data, or other data (e.g., time of day), with each other during the time period.

In certain embodiments, at block 610, calibration engine 154 determines whether the patient (e.g., user 102) is in a horizontal (i.e., lying) position. Since many compression events are caused by the patient lying on the body part on which their continuous analyte sensor is being worn, determining whether the patient is in a horizontal position may be a good indicator that a drop in first analyte levels is caused by a compression event. In such embodiments, in order to determine whether the patient is in a horizontal position, calibration engine 154 may process and correlate one or more types of the received physiological data from block 606. In specific examples, calibration engine 154 may determine whether the patient is lying down by processing and mapping (e.g., correlating) gyroscope data, accelerometer data, and/or other suitable types of secondary sensor data, with each other during the time period.

If the calibration engine 154 determines that the patient is sleeping or lying down at block 610, then method 600 proceeds to block 612. If, however, it is determined that the patient is not sleeping or not lying down, then method 600 proceeds to block 620.

At block 612, method 600 continues by calibration engine 154 determining whether a posture change of the patient occurred prior to the drop in first analyte levels. The determination that the patient changed their posture prior to the drop in first analyte levels provides a good indication that the posture change caused the patient to lie on the body part on which their continuous analyte sensor is being worn, thus applying external pressure on or around the sensor and causing a compression event. For example, the patient may have lain down to sleep or rest, and in the process, the patient had laid down on the body part on which their continuous analyte sensor was being worn. Alternatively, the patient had already been lying down, and had thereafter changed position such that they were lying on the body part on which their continuous analyte sensor was being worn.

In order to determine whether the patient changed posture, calibration engine 154 may process and correlate one or more types of the received physiological data from block 606. In specific examples, calibration engine 154 may determine whether a posture change occurred by processing and mapping (e.g., correlating) gyroscope data, accelerometer data, and/or other suitable types of secondary sensor data, with each other during the time period.

If the calibration engine 154 determines that the patient did change posture at block 612, then method 600 continues to block 614, where a suspected compression event has been determined. In certain embodiments, upon determination of a compression event, any inaccurate data resulting from the compression event may be blanked (e.g., removed), or the measured first analyte data may be corrected or compensated with measured analyte data for other analytes. In certain embodiments, delivery of a supplemental insulin dose may be inhibited upon the determination of a compression event. For example, calibration engine 154 may transmit a signal to an insulin pump (an example of medical device 208) to cause the pump to refrain from delivering a supplemental insulin dose or to override a decision to deliver a supplemental insulin. In certain embodiments, a notification or alert may be generated and provided to the patient regarding the compression event, e.g., via display 107 in **FIG. 1****,** and may further include a recommendation to the patient to change positions to relive the external pressure on or around the sensor. In further embodiments, the notification or alert may request confirmation input from the patient, or a caregiver, that a compression event did occur, or input indicating that the notification or alert was erroneous.

If at block 612 it is determined that no posture change is detected prior to the drop in first analyte levels, then method 600 continues at block 616, wherein calibration engine 154 processes the measured lactate data form block 604 and determines whether there is an rise in lactate levels corresponding with the drop in first analyte levels. Processing of the lactate measurements taken during the time period may include a determination of directional trends, which may include data such as a minimum measurement of lactate during the time period, a maximum measurement of lactate during the time period, a direction of change (e.g., upward rise or downward fall) of lactate levels during the time period, a rate of change of lactate levels during the time period, etc.

As described above, during compression events, cells near the sensor-tissue interface do not receive enough oxygen for aerobic respiration, and thus switch to anaerobic respiration to support cell metabolism, in which glucose is converted to lactate instead of CO₂. Accordingly, compression events typically result a detected spike in lactate, and determining that a corresponding rise in lactate levels has occurred during the time period may confirm the occurrence of a compression event. In certain embodiments, similar to block 608, determining a corresponding rise in lactate levels includes determining whether the lactate levels have satisfied one or more predetermined thresholds. For example, in certain embodiments, the one or more predetermined thresholds include measured lactate value thresholds, lactate rate of change thresholds, and/or time period thresholds, which may be utilized in combination.

If calibration engine 154 detects a corresponding rise in lactate levels at block 616, then method 600 proceeds to block 614 where a compression event is determined. If however, no corresponding rise in lactate levels is detected at block 616, then further processing and analysis of the measured first analyte data, measured additional analyte data, and/or received physiological data is performed by calibration engine 154 at block 618 to determine the cause of the drop in first analyte levels. In certain examples, the measured first analyte data and measured lactate data is cross-checked with one or more additional types of the received physiological data, such as accelerometer data and/or heart rate data, to confirm whether or not a compression event has occurred. Thus, cross-checking with received physiological data may rule out compression events, or even other types of physiological events that could possible cause the drop in first analyte levels.

Returning to block 610, if the calibration engine 154 determines that the patient is not sleeping or lying down, then method 600 proceeds to block 620. At block 620, calibration engine 154 determines whether the patient is exercising during the time period, or whether the patient is in a vertical (i.e., sitting or standing) position. Determining that the patient is either exercising or in a vertical position may be a good indicator that a drop in first analyte levels is not caused by a compression event, and instead, may be caused by the patient engaging in physical activity, or some other physiological event.

In certain embodiments, calibration engine 154 determines whether the patient (e.g., user 102) is exercising by processing and correlating measured analyte data for the first analyte and/or one or more additional analytes from blocks 602 and 604, in addition to one or more types of the received physiological data from block 606. In specific examples, calibration engine 154 may determine whether the patient is exercising by processing and mapping (e.g., correlating) measured analyte levels for, e.g., glucose and lactate, in addition to heart rate data, accelerometer data, respiration data, blood pressure data, and/or other suitable types of secondary sensor data, with each other during the time period.

In certain embodiments, calibration engine 154 determines whether the patient is in a vertical position by processing and correlating one or more types of the received physiological data from block 606. In specific examples, calibration engine 154 may determine whether the patient is in a vertical position by processing and mapping (e.g., correlating) gyroscope data, accelerometer data, and/or other suitable types of secondary sensor data, with each other during the time period.

If the calibration engine 154 determines that the patient is exercising or in a vertical position at block 620, then further processing and analysis of the measured first analyte data, measured additional analyte data, and/or received physiological data is performed by calibration engine 154 at block 618 to determine the cause of the drop in first analyte levels. In certain examples, the measured first analyte data and measured lactate data is cross-checked with one or more additional types of the received physiological data, such as accelerometer data and/or heart rate data, to confirm whether the drop in first analyte levels was caused by, e.g., exercise or other physiological events, or whether the drop was caused by a compression event (though unlikely).

If, however, it is determined that the patient is not exercising or in a vertical position, then method 600 proceeds to block 622, wherein calibration engine 154 processes the measured lactate data from block 604 and determines whether there is an rise in lactate levels corresponding with the drop in first analyte levels. Processing of the lactate measurements taken during the time period may include a determination of directional trends, which may include data such as a minimum measurement of lactate during the time period, a maximum measurement of lactate during the time period, a direction of change (e.g., upward rise or downward fall) of lactate levels during the time period, a rate of change of lactate levels during the time period, etc.

As described above, compression events typically result a detected spike in lactate, and determining that a corresponding rise in lactate levels has occurred during the time period, without the patient engaging in exercising or being in a vertical position, may confirm the occurrence of a compression event. In certain embodiments, similar to block 608 or 616, determining a corresponding rise in lactate levels includes determining whether the lactate levels have satisfied one or more predetermined thresholds. For example, in certain embodiments, the one or more predetermined thresholds include measured lactate value thresholds, lactate rate of change thresholds, and/or time period thresholds, which may be utilized in combination.

If calibration engine 154 detects a corresponding rise in lactate levels at block 622, then method 600 proceeds to block 614 where a compression event is determined. If, however, no corresponding rise in lactate levels is detected at block 622, then further processing and analysis of the measured first analyte data, measured additional analyte data, and/or received physiological data is performed by calibration engine 154 at block 618 to determine the cause of the drop in first analyte levels. In certain examples, the measured first analyte data and measured lactate data is cross-checked with one or more additional types of the received physiological data to confirm whether or not a compression event has occurred. For example, further analysis at block 618 may rule out the occurrence of a compression event, or any other types of physiological events that could possible cause the drop in first analyte levels.

In certain embodiments, one or more blocks of method 600 are performed utilizing one or more trained machine learning models, e.g., trained machine learning models provided by training server system 140 in **FIG. 1****,** or other AI models. For example, one or more determinations of method 600 may utilize regression models, decision tree models, neural networks such as deep neural networks, and/or fuzzy logic, as well as other types of computational models. In certain embodiments, such models may be configured to use, as input, measured data from one or more sensors measuring multiple analytes to provide, as output, determinations utilized for recalibration of a continuous analyte sensor. Example inputs for such models may, for example, include continuous analyte sensor data (e.g., for glucose, lactate, and/or any other analytes), non-analyte sensor data (e.g., gyroscope data, heart rate data, accelerometer data), time, etc. Accordingly, given the interaction of various physiological states, parameters and/or thresholds of such algorithms or models may be altered based, at least in part, on a number of analytes or characteristics being measured for input to reflect the knowledge attained from each of the other analytes or characteristics being measured or the physical states associated with the additional analytes or characteristics being measured.

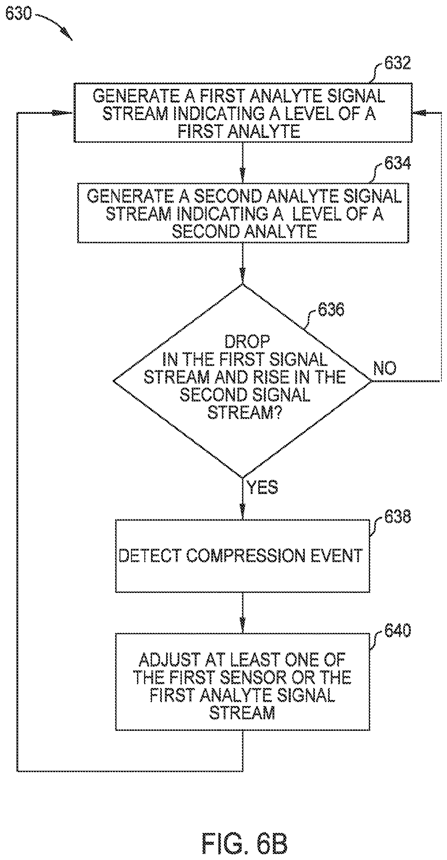

**FIG. 6B** illustrates a flow diagram depicting an example method 630 for detecting and compensating for a compression event. In certain embodiments, the continuous analyte monitoring system 104 performs the method 630. By performing the method 630, the continuous analyte monitoring system 104 detects a compression event using two different analyte signal streams.

At block 632, the continuous analyte monitoring system 104 begins by generating a first analyte signal stream indicating a level of a first analyte (e.g., glucose). The continuous analyte monitoring system 104 may use a first sensor to continuously or non-continuously monitor the first analyte of a patient, such as user 102 illustrated in **FIG. 1****.** In certain embodiments, the first sensor may be a factory-calibrated continuous glucose sensor 202. In certain other embodiments, the first sensor may be a factory-calibrated continuous multi-analyte sensor that measures at least the user's glucose levels. The first analyte signal stream may be an analyte signal that changes over time indicating changes to the level of the first analyte.

At block 634, the continuous analyte monitoring system 104 generates a second analyte signal stream indicating a level of a second analyte (e.g., lactate). The continuous analyte monitoring system 104 may use a second sensor or the first sensor to continuously or non-continuously monitor data (e.g., concentration values, trends, or patterns) from the user to obtain the second analyte signal stream. The second analyte signal stream may be an analyte signal that changes over time indicating changes to the level of the second analyte.

In certain embodiments, the continuous analyte monitoring system 104 uses the same sensor at block 634 as was used at block 632, such as a continuous analyte sensor 202. In other embodiments, the continuous analyte monitoring system 104 uses a different sensor at block 634 as at block 632.

At block 636, the continuous analyte monitoring system 104 compares the first analyte signal stream to the second analyte signal stream and monitors data trends for each signal stream. Specifically, the continuous analyte monitoring system 104 monitors rises and drops of each the first analyte signal stream and the second analyte signal stream. If there is a drop in the first analyte signal stream and a rise in the second analyte signal stream during the same time period, the continuous analyte monitoring system 104 proceeds to block 638. If there is not a drop in the first analyte signal stream and a rise in the second analyte signal stream during the same time period, the continuous analyte monitoring system 104 returns to block 632 and continues monitoring sensor data.

At block 638, the continuous analyte monitoring system 104 detects a compression event by determining that the pattern of a drop in the first analyte signal stream and a rise in the second analyte signal stream during the same time period is indicative of a compression event (e.g., the first sensor measuring the first analyte is being compressed). Compression of a sensor may result in small spikes in analyte data that are not truly representative of the concentration of the first analyte in the blood stream of the user. These spikes may lead to incorrect data presented to a medical professional or the user.

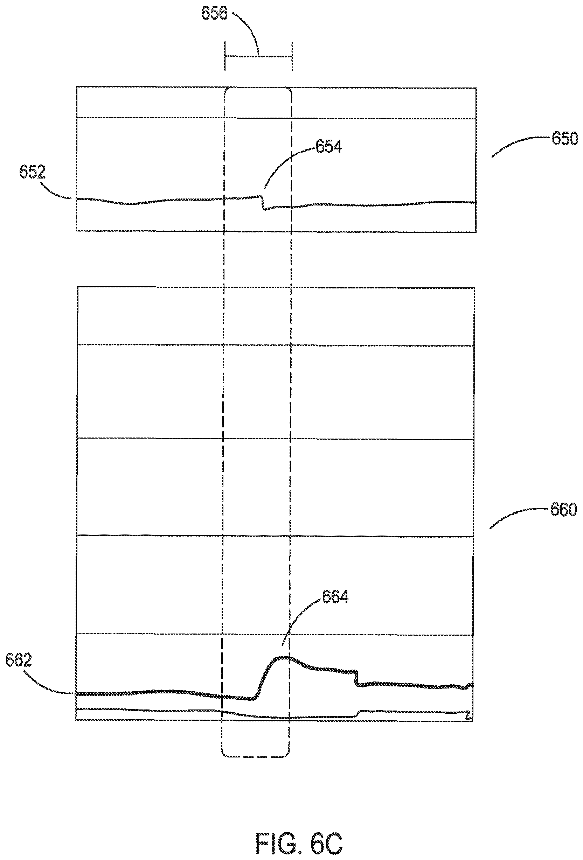

As shown in **FIG. 6C****,** a first plot 650 shows a first analyte signal stream 652, and a second plot 660 shows a second analyte signal stream 662. The first analyte signal stream 652 may experience a drop 654 during a period of time 656. A compression event is detected when, during the same period of time 656, the second analyte signal stream 662 indicates a rise 664. For example, the first analyte signal stream 652 may indicate a measured glucose level, and the second analyte signal stream 662 may indicate a measured lactate level. The continuous analyte monitoring system 104 may determine that a compression event occurred when the continuous analyte monitoring system 104 determines that a drop 654 occurred in the glucose levels during the same time period 656 that a rise 664 occurred in the lactate levels.

Returning to **FIG. 6B****,** at block 640, once a compression event is detected in block 408, the continuous analyte monitoring system 104 may then adjust at least one of the first sensor or the first analyte signal stream. In adjusting the first sensor, the continuous analyte monitoring system 104 may temporarily power down the first sensor such that the first sensor does not collect data for a pre-determined period of time. The pre-determined period of time may be a set time or may be based on data from the second analyte signal stream (e.g., when the second analyte signal stream drops back down from the rise caused by the compression). Adjusting the first analyte signal stream may be accomplished by applying a correction to the first analyte signal stream for a pre-determined period of time. The amount of correction may depend on the second analyte signal stream (e.g., when the second analyte signal stream drops back down from the rise caused by the compression). Alternatively, adjusting the first analyte signal stream may be accomplished by ignoring, purging, or not recording the first analyte signal stream for the pre-determined period of time. The pre-determined period of time may be a set time or may be based on the second analyte signal stream (e.g., when the second analyte signal stream drops back down from the rise caused by the compression).

### Dip and Recovery (DnR)

Often, upon insertion of an in vivo continuous analyte sensor, the sensor may exhibit prolonged dips in measured glucose values, which eventually recover to expected levels. This phenomena is generally referred to as "dip and recover" (DnR), and may be caused by an increased consumption of glucose for immediate energy to drive the immune response at the insertion site. Such DnR events can last anywhere between a few hours to multiple days from the time of insertion, which can lead to excessive low glucose alerts and erroneous glucose readings.

Currently, some continuous analyte monitoring systems compensate for DnR events by using empirically derived models to apply an average compensation for all patients of a population. However, because such methods are population-based, they sacrifice accuracy at the individual level. Thus, there is a need for systems, devices, and methods for automatically detecting a DnR event of a patient, and/or for automatically adjusting measurements, at the individual level, to compensate for the DnR event. Described herein are systems, devices, and methods for accomplishing the aforementioned.

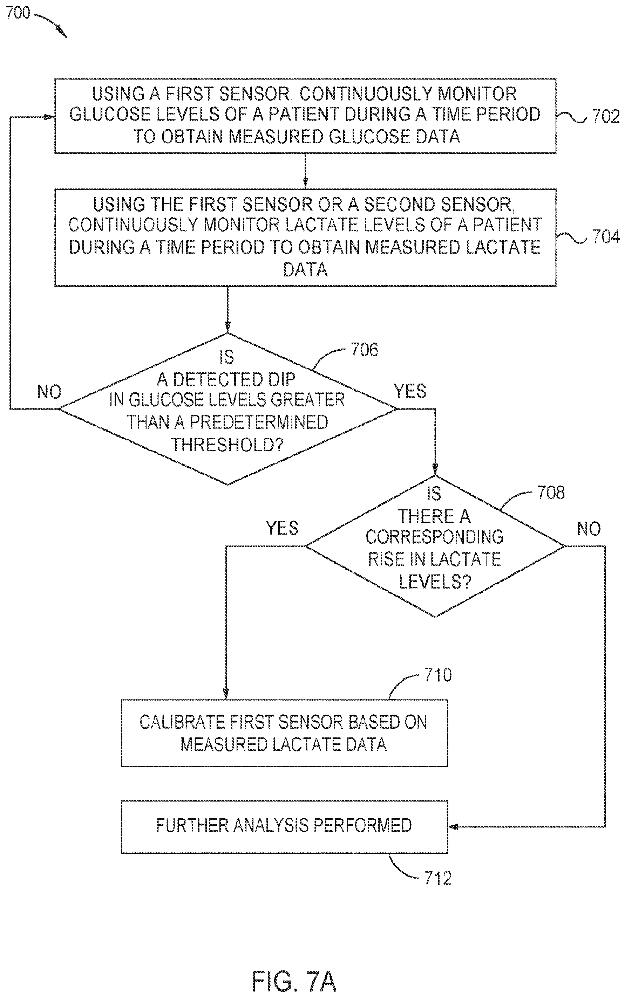

**FIG. 7A** illustrates a flow diagram of an example method 700 for determining the occurrence of a DnR event upon insertion of a continuous analyte sensor and calibrating the continuous analyte sensor based on the determined DnR event, in accordance with certain aspects of the present disclosure. Method 700 may be performed to determine a wear location for one or more continuous analyte sensors 202 of continuous analyte monitoring system 104, as illustrated in **FIGs. 1** and **2****.** Thus, method 700 is described below with reference to **FIGs. 1** and **2** and their components.

At block 702, method 700 begins by using a first, factory-calibrated sensor, which has been recently inserted onto a patient, to continuously or non-continuously monitor glucose levels of the patient, such as user 102 illustrated in **FIG. 1****,** during a time period to obtain measured glucose data. Block 702 may be performed by continuous analyte monitoring system 104 illustrated in **FIGs. 1** and **2****,** and more specifically, continuous analyte sensor(s) 202 illustrated in **FIG. 2****,** which may be glucose sensors in certain embodiments.

At block 704, method 700 continues by continuously or non-continuously monitoring lactate levels of the patient during the time period to obtain measured lactate data. Lactate levels typically exhibit the exact opposite phenomena to glucose DnR upon insertion of a continuous analyte sensor. For example, lactate signals typically rise for prolonged periods of time after sensor insertion and then eventually recover to expected levels in a manner that mirrors the DnR of glucose signals. This effect may be attributed to increased production of lactate by aerobic glycolysis via beta2 receptor stimulation, as well as reduced clearance during immunological activation and inflammation. Accordingly, due to lactate's near-opposite response to sensor insertion, lactate levels may be utilized to recalibrate or equilibrate measured glucose values, as described further below.

In certain embodiments, block 704 is performed by the same sensor as block 702, such as a continuous analyte sensor 202 (e.g., continuous analyte sensor 202 may include a continuous multi-analyte sensor configured to measure both glucose and lactate). In other embodiments, block 704 is performed a second factory-calibrated sensor different from the first sensor used in block 702.

To rule out elevated or irregular lactate levels and/or trends caused by other events (e.g., eating, exercise, emotional stress, or sepsis), the measured lactate data may be mapped against other types of data, including the levels and/or trends of other analytes as well as other, non-analyte data. For example, to rule out lactate changes caused emotional stress or other confounding causes, the measured lactate data may be compared by calibration engine 154 against non-analyte sensor data from, e.g., a physical activity sensor such as a hear rate monitor or accelerometer, which may confirm that the user is engaging in physical activity. In certain embodiments, to rule out lactate changes caused by the consumption of a meal, which may cause a slight increase in lactate and only a slight increase in glucose, calibration engine 154 may further compare the measured lactate data and/or measured glucose data against data and/or metrics associated with meal consumption by the user (e.g., metrics 130), which may confirm that the user has or has not recently consumed a meal.

At block 706, method 700 continues with calibration engine 154 processing the measured glucose data to detect the occurrence of a dip (e.g., decrease or downward fall) in glucose levels that satisfies one or more predetermined thresholds. For example, the glucose measurements taken during the time period are processed to determine directional trends, which may include data such as a minimum measurement of glucose during the time period, a maximum measurement of glucose during the time period, a direction of change (e.g., upward rise or downward fall) of glucose levels during the time period, a rate of change of glucose levels during the time period, etc.

In certain embodiments, the one or more predetermined thresholds include concentration thresholds. For example, in certain embodiments, the one or more predetermined thresholds include a minimum glucose concentration threshold, which may be satisfied upon the patient's measured glucose values substantially equaling, hovering around, or falling below the minimum glucose concentration threshold. In certain embodiments, the one or more predetermined thresholds include rate of change thresholds. For example, in certain embodiments, the one or more predetermined thresholds include a maximum glucose rate of change threshold, which may be satisfied upon the patient's measured glucose values declining at a rate substantially equal to or greater than the maximum glucose rate of change threshold.

In certain embodiments, the one or more predetermined thresholds include time period thresholds, which may be utilized in combination with one or more concentration thresholds and/or rate of change thresholds. For example, in certain embodiments, the one or more predetermined thresholds include a maximum time period threshold, which may be satisfied upon the patient's measured glucose values substantially equaling, hovering around, or falling below the minimum glucose concentration threshold, and/or the patient's measured glucose values declining at a rate substantially equal to or greater than the maximum glucose rate of change threshold, for a time period substantially equal to or greater than the maximum time period threshold.

If the detected dip in glucose levels does not satisfy the one or more predetermined thresholds, then the continuous monitoring of glucose at block 702 continues. If, however, the detected dip in glucose levels does satisfy the one or more predetermined thresholds, then at block 708, calibration engine 154 processes the measured lactate data and determines whether there is a rise in lactate levels corresponding to the detected dip in glucose levels. Processing of the lactate measurements taken during the time period may include a determination of directional trends, which may include data such as a minimum measurement of lactate during the time period, a maximum measurement of lactate during the time period, a direction of change (e.g., upward rise or downward fall) of lactate levels during the time period, a rate of change of lactate levels during the time period, etc.

As described above, lactate levels typically exhibit opposite phenomena to glucose DnR upon insertion of a continuous analyte sensor. Thus, determining that a corresponding rise in lactate levels has occurred during the time period may confirm the existence of a simultaneous DnR event. In certain embodiments, similar to block 706, determining a corresponding rise in lactate levels includes determining whether the lactate levels have satisfied one or more predetermined thresholds. For example, in certain embodiments, the one or more predetermined thresholds include measured lactate value thresholds, lactate rate of change thresholds, and/or time period thresholds, which may be utilized in combination.

If calibration engine 154 detects a corresponding rise in lactate levels at block 708, then at block 710, calibration engine 154 may recalibrate the first sensor based at least partially on the measured lactate data. In other words, the calibration engine 114 may be configured to convert at least one sensor data point received from the first sensor for glucose into recalibrated data, in real time, based at least in part on the measured lactate data. In certain embodiments, calibration engine 154 determines one or more correction factors for glucose measurements taken by the first sensor. In certain embodiments, the one or more correction factors are based at least partially on predetermined historical mappings of glucose and lactate data. In certain embodiments, the one or more correction factors may generally include an adjustment to sensitivity (m), rate of change of sensitivity (ddm/ddt), baseline/intercept (b), rate of change of baseline (ddb/ddt), sensitivity profile, relationships between particular stimulus signal outputs (e.g., impedance) to sensor sensitivity, relationships between particular stimulus signal outputs to sensor temperature or temperature, and/or calibration code associated with the first sensor. In certain embodiments, a plurality of possible values for each of the one or more correction factors may be determined, to which a weighting or averaging function may be applied. In certain embodiments, new values may be determined for one or more sensor calibration parameters (e.g., sensitivity (m), baseline/intercept (b), etc.) without an adjustment of a prior known calibration parameter via one or more correction factors during recalibration.

If however, no corresponding rise in lactate levels is detected at block 708, then further processing and analysis of the measured glucose data is performed by calibration engine 154 at block 712 to determine the cause of the dip in glucose levels. For example, such analysis may result in a prediction that the patient is, or will be, experiencing, e.g., a glycemic or other physiological event.

Although method 700 is described above with regards to the utilization of lactate for equilibration of glucose DnR events, other sensor data, including other measured analyte data and/or secondary sensor data, may be used in combination with or alternatively to measured lactate data for DnR compensation. For example, in certain embodiments, glutamate may be monitored to detect DnR events, as well as end-of-life events of continuous analytes sensors. Typically, upon insertion of an in vivo continuous analyte sensor into a tissue, the surrounding tissue environment initiates an injury response that forms a physical diffusion barrier around the insertion site to prevent diffusion of foreign bodies into the patient's body. In certain cases, the physical diffusion barrier prevents glutamate, and by extension, glucose, from freely accessing the recently-inserted sensor. Thus, because interstitial glutamate levels are maintained within a known physiological range for a given patient, sensor glutamate readings (e.g., electrochemical sensors utilizing glutamate oxidase and/or glutamate dehydrogenase enzymes to detect interstitial glutamate) can be monitored to identify if an injury response has formed a diffusion barrier, thereby preventing glutamate and glucose from accessing the sensor and causing a DnR-type event. Accordingly, measured glutamate data may be utilized to identify or confirm the occurrence of a DnR event, and further be utilized to equilibrate glucose sensor readings.

In still further embodiments, in addition to lactate, measured analyte data for one or more additional analytes may be utilized in detecting and/or equilibrating glucose DnR events. For example, in certain embodiments, at blocks 706 and/or 708, calibration engine 154 may further determine the presence of a glucose DnR event based on mappings or correlations between the measured glucose data, the measured lactate data, and/or secondary sensor data. Examples of secondary sensor data include accelerometer data, gyrometer data, global positioning system (GPS) data, heart rate, heart rate reserve, heart rate variability (HRV), electrocardiogram (EKG) data, electromyogram (EMG), respiration rate, temperature, blood pressure, galvanic skin response, oxygen uptake data (e.g., VO₂ max), sleep, impedance data, strain sensor data, etc. In certain embodiments, at blocks 706 and/or 708, calibration engine 154 may further determine the presence of a glucose DnR event based on mappings or correlations between the measured glucose data, the measured lactate data, and/or measured analyte data for one or more additional analytes, such as ketones, glycerol, potassium (e.g., hyperkalemia), sodium, CO₂ or anion-gap, or similar analytes.

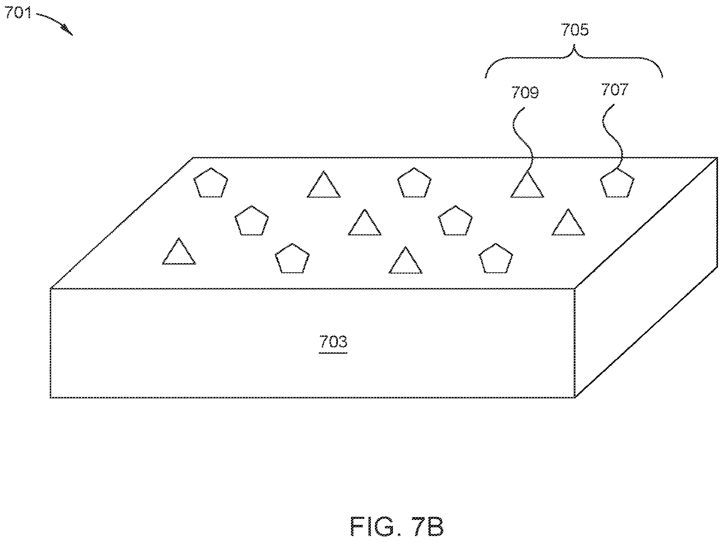

**FIG. 7B** schematically illustrates a portion of a sensing region 701 of a continuous glucose sensor, in accordance with certain aspects of the present disclosure. Sensing region 701 is configured to facilitate compensation for DnR events of measured glucose readings upon the initial insertion of the continuous multi-analyte sensor into a tissue of a patient. Generally, sensing region 701 may be used with any suitable continuous glucose sensors, such as continuous analyte sensors 202 of continuous analyte monitoring system 104, as illustrated in **FIGs. 1** and **2****.** In certain embodiments, sensing region 701 may be utilized with a continuous multi-analyte sensor.

As illustrated, sensing region 701 comprises an enzymatic membrane 703 having an enzyme matrix 705 comprised of a predetermined quantity of immobilized lactate or lactose oxidase (LO) enzyme 709, in addition to glucose oxidase (GO) enzyme 707. The addition of the LO enzyme 709 in enzyme matrix 705 facilitates measurable oxidation of lactate or lactose in the sensing region 701, which compensates for reduced glucose oxidation by the GO enzyme 707 during a DnR event, thus creating a more leveled glucose signal during the DnR event. The quantity of LO enzyme 709 loaded onto the enzyme matrix 705 is sufficient enough to affect glucose sensor readings for a limited amount of time, after which the LO enzyme 709 will have greatly reduced or no enzymatic activity. For example, in certain embodiments, the quantity of LO enzyme 709 loaded onto the enzyme matrix 705 is sufficient to affect the glucose sensor readings for about 5 or less days, or about 4 or less days, or about 3 or less days, or about 2 or less days, or about 1 or less days.

Accordingly, in addition to or alternatively to the method 700 described above, the utilization of a continuous glucose sensor having a working electrode with sensing region 701 may facilitate improved glucose sensor readings during DnR events, as the addition of the LO enzyme 709 to the sensing regions provides an offsetting effect for the reduced glucose signal.

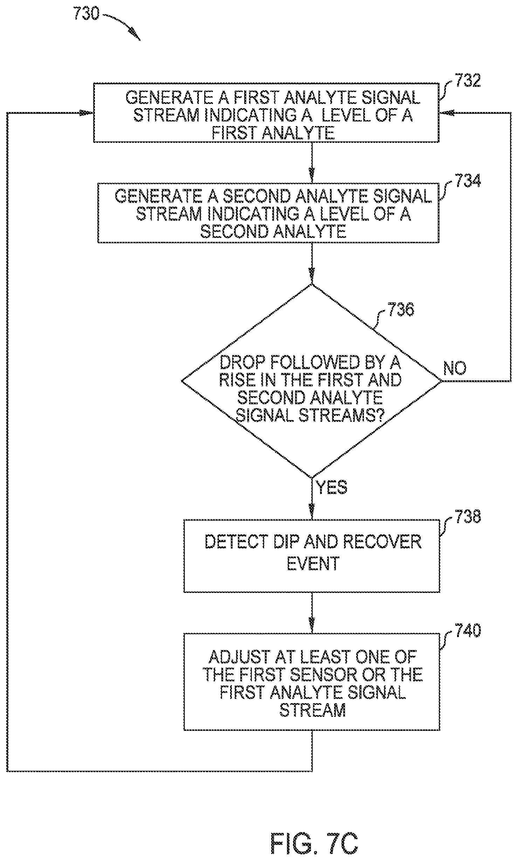

**FIG. 7C** illustrates a flow diagram of an example method 730 for identifying sensor dip and recover events, in accordance with certain aspects of the present disclosure. In certain embodiments, the continuous analyte monitoring system 104 may perform the method 730 to detect and compensate for dip and recover events.

At block 732, the continuous analyte monitoring system 104 begins by generating a first analyte signal stream indicating a level of a first analyte (e.g., glucose). The continuous analyte monitoring system 104 may use a first analyte sensor to continuously or non-continuously monitor the first analyte of a patient, such as user 102 illustrated in **FIG. 1****.** The continuous analyte monitoring system 104 generates a first analyte signal stream indicating a concentration or level of the first analyte. In certain embodiments, the first sensor may be a factory-calibrated continuous glucose sensor 202. In certain other embodiments, the first sensor may be a factory-calibrated continuous multi-analyte sensor that measures at least the user's glucose levels. The first analyte signal stream may be a signal that changes over time to indicate changes in the concentration or level of the first analyte.

At block 734, the continuous analyte monitoring system 104 continues by generating a second analyte signal stream indicating a level of a second analyte (e.g., lactate). The continuous analyte monitoring system 104 may use a second analyte sensor or the first analyte sensor to continuously or non-continuously monitor data (e.g., concentration values, trends, or patterns) from the user to generate the second analyte signal stream that is indicative of a concentration or level of the second analyte. The second analyte signal stream may be a signal that changes over time to indicate changes in the concentration or level of the second analyte.

In certain embodiments, the continuous analyte monitoring system 104 uses the same sensor at block 734 as at block 732. In other embodiments, the continuous analyte monitoring system 104 uses a different sensor at block 734 as at block 732.

At block 736, the continuous analyte monitoring system 104 compares the first analyte signal stream to the second analyte signal stream and monitors data trends for each signal stream. Specifically, the continuous analyte monitoring system 104 monitors rises and drops in each the first analyte signal stream and the second analyte signal stream. If there is a drop in the first analyte signal stream and a drop in the second analyte signal stream during the same period of time followed by a rise in both the first analyte signal stream and the second analyte signal stream, the continuous analyte monitoring system 104 proceeds to block 738. Otherwise, the continuous analyte monitoring system 104 returns to block 732 and continues monitoring sensor data. In some embodiments, the rise in the second analyte signal stream occurs faster than the rise in the first analyte signal stream.

At block 738, the continuous analyte monitoring system 104 detects a dip and recover event by determining that the pattern of drops in the first analyte signal stream and the second analyte signal stream followed by rises in both signal streams is indicative of a dip and recover event, particularly if the first and second analyte sensors are initially being used (e.g., if the first analyte sensor and the second analyte sensor were first inserted into the user's body). Dip and recover events often occur during the initial use of the continuous analyte monitoring system 104, and may produce incorrectly measured analyte data.

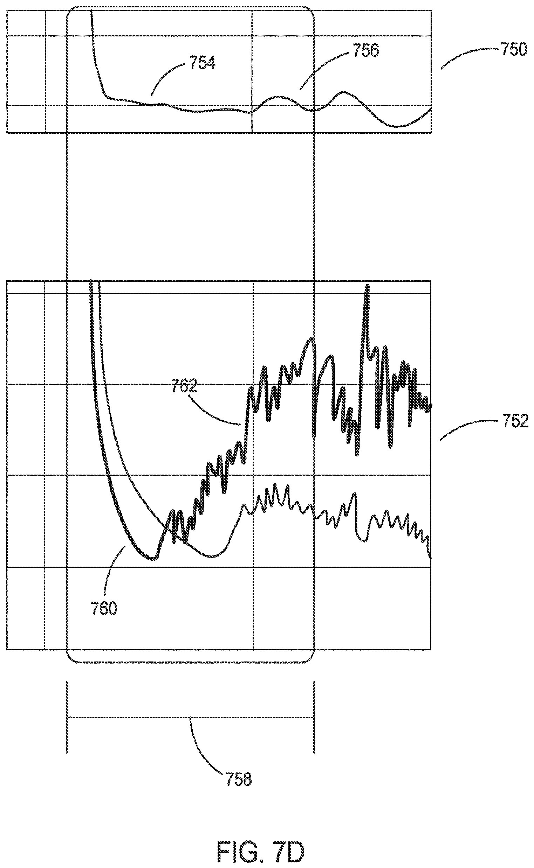

As shown in **FIG. 7D****,** a first analyte signal stream 750 and a second analyte signal stream 752 are plotted. The first analyte signal stream 750 may show a drop 754 and a gradual recovery 756 during a period of time 758. A dip and recover event is detected when the second analyte signal stream 752 shows a drop 760 followed by a recovery 762 during the same period of time 758. Notably, the recovery 762 of the second analyte signal stream 752 may occur faster and to a greater degree than the recovery 756 of the first analyte signal stream 750.

Returning to **FIG. 7C****,** at block 740, after a dip and recover event is detected in block 738, the continuous analyte monitoring system 104 may adjust at least one of the first analyte sensor or the first analyte signal stream. In adjusting the first analyte sensor, the continuous analyte monitoring system 104 may temporarily power down the first analyte sensor such that the first analyte sensor does not collect data for a pre-determined period of time. The pre-determined period of time may be a set time or may be based on data from the second analyte signal stream (e.g., when the second analyte signal stream finishes rising and recovering from the dip and recover event). Adjusting the first analyte signal stream may be accomplished by applying a correction to the first analyte signal stream for a pre-determined period of time. The amount of the correction may depend on the second analyte signal stream (e.g., when the second analyte signal stream finishes rising and recovering from the dip and recover event). Alternatively, adjusting the first analyte signal stream may be accomplished by ignoring or not recording the first analyte signal stream for the pre-determined period of time. The pre-determined period of time may be a set time or may be based on data from the second analyte signal stream (e.g., when the second analyte signal stream finishes rising and recovering from the dip and recover event).

### Hypoglycemic Events

A user may experience a hypoglycemic event when the user's glucose levels are too low. It may be not be sufficient or accurate, however, to detect hypoglycemic events based solely on measured glucose concentrations or levels, especially at night when the user is sleeping. For example, some measured glucose levels may be inaccurate due to compression events that occur during sleep.

The continuous analyte monitoring system 104 may perform some additional checks when a measured glucose concentration or level is too low to verify whether a hypoglycemic event is actually occurring. For example, the continuous analyte monitoring system 104 may check whether the user is sleeping. The continuous analyte monitoring system 104 may determine the time of day to see if the glucose concentration or level was measured at night or at a time when the user is typically sleeping. Additionally, the continuous analyte monitoring system 104 may use an accelerometer reading (e.g., using an accelerometer in the continuous analyte monitoring system 104) to determine whether the user's movement is consistent with sleep, sleep apnea, or compression (e.g., using some of the processes shown using **FIG. 6A****).** As another example, the continuous analyte monitoring system 104 may check a measured local tissue oxygen saturation (e.g., using an oxygen sensor of the continuous analyte monitoring system 104) to determine if a compression event is occurring. If the oxygen level is too low in that region, then the continuous analyte monitoring system 104 may determine that compression is occurring, which may negatively impact that accuracy of the glucose measurement. As another example, the continuous analyte monitoring system 104 may check a measured lactate concentration or level within a similar timeframe as the glucose measurement. If the lactate concentration did not increase around the time the glucose concentration or level was measured, the continuous analyte monitoring system 104 may determine that the glucose measurement was a false reading. After performing the checks, if the continuous analyte monitoring system 104 determines that the measured glucose concentration or level was accurate, the continuous analyte monitoring system 104 may issue an alert indicating the detected hypoglycemic event.

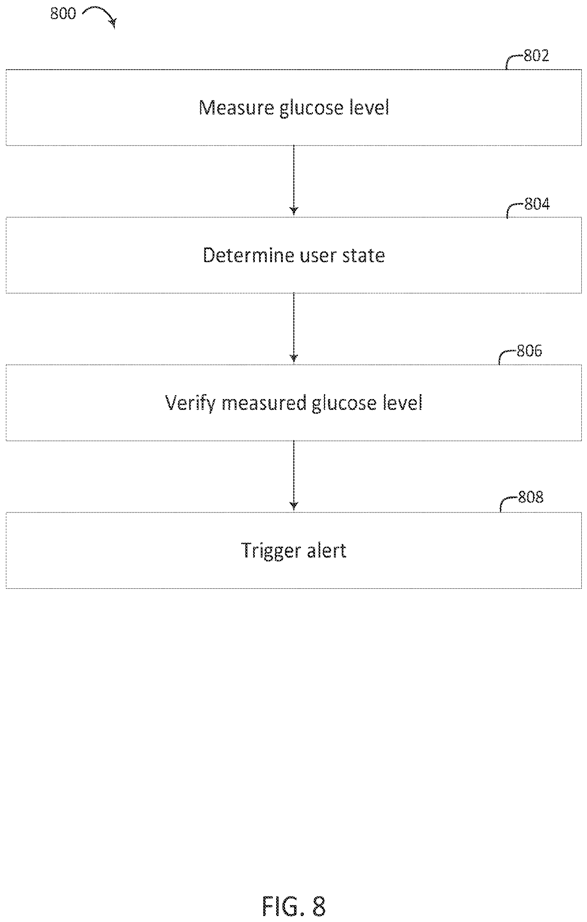

**FIG. 8** illustrates a flow diagram of an example method 800 for verifying a hypoglycemic event. In certain embodiment, the continuous analyte monitoring system 104 performs the method 800. By performing the method 800, the continuous analyte monitoring system 104 performs additional checks using other sensors before concluding that a hypoglycemic event is occurring.

At block 802, the continuous analyte monitoring system 104 measures a glucose level of a user. The continuous analyte monitoring system 104 may use a glucose sensor of the continuous analyte monitoring system 104 to measure the glucose level of the user after the user has inserted the glucose sensor into the user's body. The continuous analyte monitoring system 104 may determine that the measured glucose level is too low, indicating a hypoglycemic event. The continuous analyte monitoring system 104 may analyze information from other sensors before concluding that a hypoglycemic even is occurring.

At block 804, the continuous analyte monitoring system 104 may determine a user state. For example, the continuous analyte monitoring system 104 may use a time of day and accelerometer readings (e.g., from an accelerometer of the continuous analyte monitoring system 104) to determine whether the user is sleeping. As another example, the continuous analyte monitoring system 104 may determine a local tissue oxygen saturation level (e.g., from an oxygen sensor of the continuous analyte monitoring system 104). As yet another example, the continuous analyte monitoring system 104 may determine a lactate concentration or level for the user (e.g., from a lactate sensor of the continuous analyte monitoring system 104).

At block 806, the continuous analyte monitoring system 104 may verify the measured glucose level. For example, the continuous analyte monitoring system 104 may determine from the time of day and the accelerometer readings that the user is not sleeping, which indicates that the measured glucose level is accurate. As another example, the continuous analyte monitoring system 104 may determine from the local tissue oxygen saturation level that a compression event is not occurring that would affect the accuracy of the glucose measurement. As yet another example, the continuous analyte monitoring system 104 may determine from the lactate measurement that the glucose measurement is not a false reading (e.g., that the lactate measurement increased within a similar timeframe of the glucose measurement). In response to one or more of these checks, the continuous analyte monitoring system 104 may verify that the glucose measurement was accurate.

At 808, the continuous analyte monitoring system 104 may trigger an alert indicating a hypoglycemic event. The alert may inclue a message, an audible sound, or a vibration that indicates to the user wearing the continuous analyte monitoring system 104 that the hypoglycemic event has been detected.

### Determining Sensor Wearing Location and Compensation

Although wearable in vivo continuous analyte sensors are typically recommended to be worn on one portion of the body (e.g., the abdomen), many patients prefer wearing their devices on other parts of the body, such as the inner arm, forearm, thigh, calf, back, or even buttock. However, sensor signal accuracy and/or quality of signal (e.g., presence of signal noise) may differ slightly between these different wear locations, and especially between the abdomen and arms. For example, a continuous glucose sensor may be more accurate when worn on the arms as compared to the abdomen, but may be prone to signal noise and earlier working failure due to frequent movement of the arms, which causes mechanical interferences. Such differences in signal accuracy or quality between the different wear locations may also be explained by temperature compensations (e.g., differences in temperature) between the body parts.

In light of the above, there is a need for improved systems and methods for automatically detecting a wear location of a wearable in vivo continuous analyte sensor on a patient, and for automatically recalibrating or adjusting measurements according to the determined wear location. Described herein are systems and methods for accomplishing such.

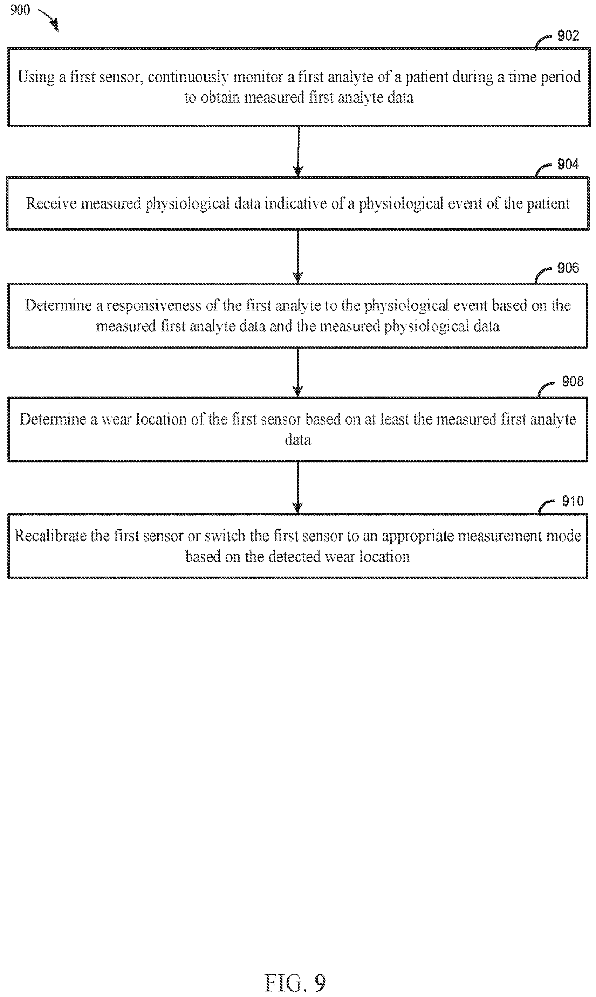

**FIG. 9** illustrates a flow diagram of an example method 900 for determining a wear location of a continuous analyte sensor and calibrating the continuous analyte sensor based on the determined wear location, in accordance with certain aspects of the present disclosure. Method 900 may be performed to determine a wear location for one or more continuous analyte sensors 202 of continuous analyte monitoring system 104, as illustrated in **FIGs. 1** and **2****.** Thus, method 900 is described below with reference to **FIGs. 1** and **2** and their components.

At block 902, method 900 begins by using a first, factory-calibrated sensor to continuously or non-continuously monitor a first analyte of a patient, such as user 102 illustrated in **FIG. 1****,** during a time period to obtain measured analyte data for the first analyte. The first sensor may generally be inserted at a desired location by the patient, such as the arm, abdomen, back, thigh, etc., and the location will be determined and compensated for by the current method.

Block 902 may be performed by continuous analyte monitoring system 104 illustrated in **FIGs. 1** and **2****,** and more specifically, continuous analyte sensor(s) 202 illustrated in **FIG. 2****,** in certain embodiments. For example, continuous analyte monitoring system 104 may comprise a factory-calibrated continuous glucose sensor 202 to measure the user's glucose levels. Alternatively, continuous analyte monitoring system 104 may comprise a factory-calibrated continuous multi-analyte sensor configured to measure at least the user's glucose levels. Generally, the first analyte may include glucose, though other analytes such as lactate, ketones, glycerol, electrolytes such as sodium and potassium, calculated measurements such as anion gap, or any other suitable analytes described herein, are also contemplated.

At block 904, method 900 continues by optionally receiving measured physiological data associated with the patient during the time period. The measured physiological data may include other sensor data measured continuously or non-continuously by one or more other sensors which may, in certain embodiments, be indicative of a physiological event of the patient during the time period. For example, the measured physiological data may indicate the onset, existence, and/or conclusion of a physiological event of the patient. In certain embodiments, the physiological data may indicate one or more physiological events of the patient during the time period. Generally, the physiological event of the patient may refer to any event or activity, experienced or engaged by the patient, that causes physical stress(es) on the patient. Examples of physiological events may include the engagement in physical activity, sleep or rest, the consumption of a meal, the administration of a medication, the occurrence of a glycemic event (e.g., hypo- or hyperglycemia), etc.

The measured physiological data may be provided by a variety of different types of sensors, either alone or in combination. In certain embodiments, the measured physiological data received at block 904 includes measured analyte data for one or more additional analytes, including at least a second analyte of the patient, during the time period. For example, in certain embodiments, the measured physiological data includes measured analyte data (e.g., concentration values, trends, and/or patterns) for lactate, ketones, glycerol, electrolytes such as sodium and potassium, calculated measurements such as anion gap, and/or any other suitable analytes described herein or combinations thereof.

In certain embodiments, the measured physiological data received at block 904 includes secondary sensor data measured during the time period, such as one or more of accelerometer data, gyrometer data, global positioning system (GPS) data, heart rate, heart rate reserve, heart rate variability (HRV), electrocardiogram (EKG) data, electromyogram (EMG), respiration rate, temperature, sweat, blood pressure, galvanic skin response, oxygen uptake data (e.g., VO₂ max), sleep, impedance data, etc. In certain embodiments, the measured physiological data may be utilized to calculate metrics, such as metrics 130 in **FIG. 1****,** to further inform the determination of wear location and compensation therefor.

In certain embodiments, the measured physiological data at block 904 is received from the same sensor utilized in block 902, such as a continuous analyte sensor 202 of continuous analyte monitoring system 104 illustrated in **FIGs. 1** and **2** (e.g., continuous analyte sensor 202 may include a continuous multi-analyte sensor configured to measure both a second analyte and the first analyte). In other embodiments, the measured physiological data at block 904 is received from a second sensor different than the first sensor used in block 902.

At block 906, method 900 continues by calibration engine 154 optionally processing the measured first analyte data and the received physiological data to determine a responsiveness of the first analyte to the indicated physiological event, based on the measured first analyte data and the measured physiological data during the time period. The responsiveness of the first analyte may refer to a degree of change, delay in change, and/or rate of change of the measured first analyte values in relation to and as effected by the indicated physiological event. For example, the engagement of the patient in physical activity may cause a decline in the patient's measured glucose levels, and the amount of decline, delay between the start of the physical activity and the measured decline, and/or the rate of decline may be referred to as the "responsiveness" of the patient's glucose levels. Generally, the responsiveness of an analyte to a physiological event varies at different wearing locations (e.g., certain analytes may exhibit more responsiveness at the arm due to proximity of the arm to the heart). Thus, after determining the responsiveness of the first analyte to the indicated physiological event, the responsiveness may be utilized to determine the wearing location of the first sensor.

At block 908, method 900 continues by calibration engine 154 determining a wear location of the first sensor based at least partially on the measured first analyte data during the time period, and in certain embodiments, the received physiological data during the time period. In certain embodiments, the wear location is determined utilizing predetermined mappings (i.e., correlations) of historical (i.e., previously measured) analyte data for the first analyte and historical physiological data of the patient and/or the general population.

In certain embodiments, the wear location of the first sensor is determined utilizing one or more trained machine learning models, e.g., trained machine learning models provided by training server system 140 in **FIG. 1****,** or other AI models, in which the historical analyte data for the first analyte and the historical physiological data have been input as training data. In certain embodiments, the expected analyte data for the first analyte is determined utilizing one or more trained machine learning models, e.g., trained machine learning models provided by training server system 140 in **FIG. 1****,** or other AI models. In certain embodiments, such models may be configured to use, as input, measured data from one or more sensors measuring multiple analytes to provide, as output, determinations utilized for recalibration of a continuous analyte sensor. Example inputs for such models may, for example, include continuous analyte sensor data (e.g., for glucose, lactate, and/or any other analytes), non-analyte sensor data (heart rate data, accelerometer data, gyrometer data, etc.), and the like. Accordingly, given the interaction of various physiological states, parameters and/or thresholds of such algorithms or models may be altered based, at least in part, on a number of analytes or characteristics being measured for input to reflect the knowledge attained from each of the other analytes or characteristics being measured or the physical states associated with the additional analytes or characteristics being measured.

In certain embodiments, the wear location of the first sensor is determined based on the measured first analyte data during the time period in combination with a plurality of secondary sensor data types. For example, in certain embodiments, in addition to the measured first analyte data, two or more of temperature data, walking gait data, and impedance data from a temperature sensor, accelerometer, and impedance sensor, respectively, may be utilized to determine the wear location of the first sensor, since each of these parameters may differ between wear locations. However, other secondary sensor data types, and/or combinations thereof, are also contemplated.

In certain embodiments, the wear location of the first sensor is determined based on the responsiveness of the measured first analyte data to the indicated physiological event determined at block 906. For example, it has been shown for lactate and other analytes that sensors placed on an upper body wear location (e.g., arms) may have faster response rates as compared to sensors placed on a lower body wear location (e.g., abdomen, legs). Accordingly, in such embodiments, the wear location may be determined by comparison of the determined responsiveness of the measured first analyte to historical responsiveness data for the first analyte at one or more wear locations (e.g., historical mappings or correlations between the wear location(s) and responsiveness of the first analyte thereat). Such historical responsiveness data may include the patient's own historical data and/or population-based historical data. In certain embodiments, the historical responsiveness data may be input into one or more trained machine learning models, e.g., trained machine learning models provided by training server system 140 in **FIG. 1****,** or other AI models, to determine the current wear location of the first sensor, and the wear location determination could be probabilistic or categorical.

In certain embodiments, the wear location is determined based on the circadian rhythm of the measured first analyte data and the received physiological data, and the time period includes at least a 24 hours. For example, in embodiments where the received physiological data includes measured second analyte data measured by the same sensor as the first analyte, the measured analyte data for both analytes may be analyzed to determine a day-night pattern for each analyte. Thereafter, the wear location may be determined by comparison of the determined day-night patterns for the first and second analytes to historical day-night patterns of the first and second analytes at one or more wear locations. In certain embodiments, the historical day-night patterns may be utilized to train one or more trained machine learning models or other AI models, which in turn may be deployed to determine the current wear location of the first sensor.

In certain embodiments, in addition to determining the wear location of the first sensor at block 908, calibration engine 154 may determine a tissue composition at the wear location. For example, calibration engine 154 may determine a composition of muscle and fat at the wear location, which may be determined based at least partially on the determination of the wear location, and/or secondary sensor data such as impedance data. The determined tissue composition may then be utilized at block 910 for measurement adjustment and/or recalibration of the first sensor, since tissue composition may affect sensor signal accuracy and quality. Accordingly, determining the tissue composition at the wear location may facilitate improved calibration of the first sensor and/or improved accuracy of sensor readings.

In certain embodiments, the determination of the wear location is based on secondary sensor data, in addition to or alternatively to the measured first analyte data and/or physiological data. More particularly, in certain embodiments, the wear location may be determined based on accelerometer data, either alone or in combination with the measured first analyte data and/or physiological data. For example, the wear location may be delineated based on a motion of the wear location as determined by the accelerometer (e.g., a sensor on the abdomen may sense an up/down motion indicating the wear location to be the abdomen, while a sensor on the arm may sense a swinging motion indicating the wear location to be the arm).

After determining the wear location of the first sensor, then at block 910, calibration engine 154 may switch the first sensor and/or any corresponding systems to an appropriate analyte measurement mode or recalibration mode that is specific to the determined wear location, and/or recalibrate the first sensor. In other words, calibration engine 154 may switch to a location-specific algorithm and/or use of location-specific calibration parameters for measuring analyte levels that compensates for the wear location, and/or a location-specific algorithm for recalibration of the first sensor, with unique location-specific compartment/sensitivity models. In certain embodiments, calibration engine 154 optimizes or reoptimizes model parameters to best fit data received from a determined wear location.

### Training Machine Learning Models

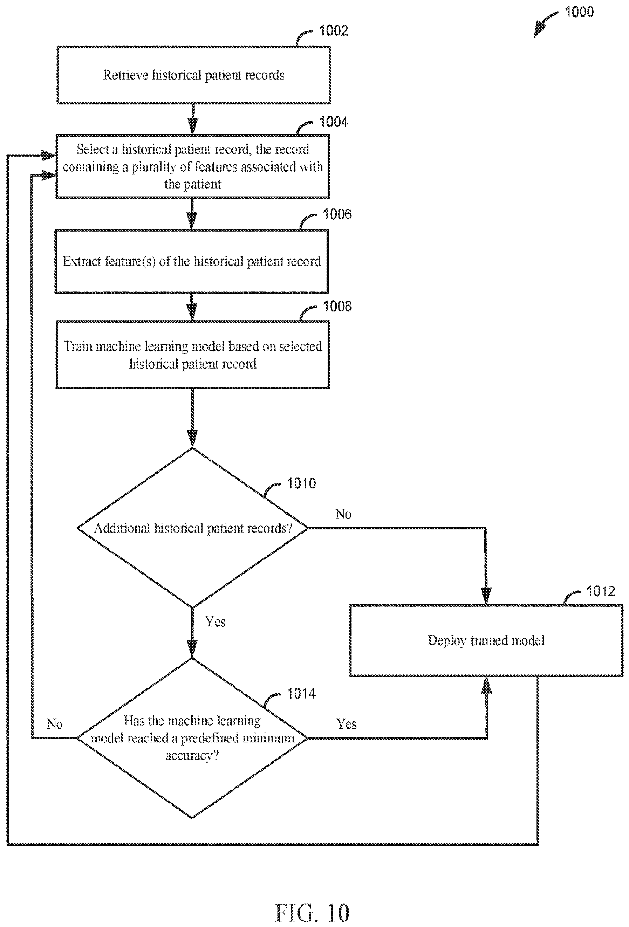

**FIG. 10** is a flow diagram depicting a method 1000 for training machine learning models to provide determinations for the sensor recalibration and measurement compensation methods described above, according to certain embodiments of the present disclosure. In certain embodiments, the method 1000 is used to train any of the machine learning models discussed herein for use in recalibration or sensor measurement compensation of continuous analyte sensor 202 of a patient, e.g., a user illustrated in **FIGs. 1-2****.**

Method 1000 begins, at block 1002, by a training server system, such as training server system 140 illustrated in **FIG. 1****,** retrieving data from a historical records database, such as historical records database 112 illustrated in **FIG. 1****.** As mentioned herein, in certain embodiments, records database 112 may provide a repository of up-to-date information and historical information for users of a continuous analyte monitoring system and connected mobile health application, such as users of continuous analyte monitoring system 104 and application 106 illustrated in **FIG. 1****,** as well as data for one or more patients who are not, or were not previously, users of continuous analyte monitoring system 104 and/or application 106. In certain embodiments, historical records database 112 may include historical data or patient-specific information associated with the patient for whom the model is being used in real-time.

Retrieval of data from historical records database 112 by training server system 140, at block 1002, may include the retrieval of all, or any subset of, information maintained by historical records database 112. For example, where historical records database 112 stores information for 100,000 patients (e.g., non-users and users of continuous analyte monitoring system 104 and application 106), data retrieved by training server system 140 to train one or more machine learning models may include information for all 100,000 patients or only a subset of the data for those patients, e.g., data associated with only 50,000 patients or only data from the last ten years.

As an illustrative example, at block 1002, training server system 140 may retrieve information for 100,000 patients stored in historical records database 112 to train a model to provide a determination utilized in the recalibration or sensor measurement compensation of a continuous analyte sensor. Each of the 100,000 patients may have a corresponding data record, such as user profile 118 illustrated in **FIG. 1****,** stored in historical records database 112. Each data record may include information, such as information discussed with respect to **FIG. 1****.**

At block 1004, method 1000 continues by training server system 140 selecting one of the historical patient records retrieved by training server system 140 at block 1002. The record contains information associated with the patient, such as the information stored in the patient's user profile. Examples of types of information included in a patient's user profile were provided above. Training server system 140 may use any suitable criteria (e.g., beginning with the oldest records, beginning with the most recent records, and the like) for selection of a historical patient record, as training server system 140 will iterate through each historical access record in the training set until all records have been used to train the machine learning model or the machine learning model is accurately determinations for recalibration and/or sensor measurement compensation for each historical patient record input into the model.

At block 1006, method 1000 continues by training server system 140 extracting one or more features of the selected historical patient record. These features are extracted to be used as input features for the machine learning model(s). For example, a user profile associated with the patient selected at block 1004 may include at least, information related to an age of the patient, a gender of the patient, normal glucose ranges of the patient, normal lactate ranges (e.g., lactate baseline and/or peak) of the patient, normal ranges for other analytes and/or other secondary sensor data of the patient, etc. Features used to train the machine learning model(s) may vary in different embodiments.

At block 1008, method 1000 continues by training server system 140 training one or more machine learning models based on the selected historical patient record. In some embodiments, the training server does so by providing the features (e.g., extracted at block 1006) as input into a model. This model may be a new model initialized with random weights and parameters, or may be partially or fully pre-trained (e.g., based on prior training rounds). Based on the input features, the model-in-training generates some output. The output may include determinations for recalibration and/or sensor measurement compensation, or similar metrics.

In certain embodiments, training server system 140 compares this generated output with the actual label associated with the historical patient record to compute a loss based on the difference between the actual result and the generated result. This loss is then used to refine one or more internal weights and parameters of the model (e.g., via backpropagation) such that the model learns to make calibration-related determinations more accurately.

At block 1010, method 1000 continues by training server system 140 determining whether additional training is needed. This may include evaluating whether any additional historical patient records remain in the training data set. Where at block 1010, training server system 140 determines all training data has been input into the machine learning model, at block 1012, training server system 140 deploys the trained model(s) during runtime. In some embodiments, this includes transmitting some indication of the trained model(s) (e.g., a weights vector) that can be used to instantiate the model(s) on another device. For example, training server system 140 may deploy the trained model(s) to calibration engine 154. The models can then be utilized for recalibration of a sensor of a user using application 106.

Where at block 1010, training server system 140 determines that not all historical patient records of the training data have been input into the model for training, at block 1014, training server system 140 determines whether the model has reached a predefined minimum accuracy (e.g., 90% accuracy, 95% accuracy, etc.). Where the predefined minimum accuracy has not been met, training server system 140 determines additional training remains, and method 1000 returns to block 1004. Alternatively, where the machine learning model satisfies the predefined minimum accuracy (e.g., 90% or 95% of the time making determinations accurately), at block 1012, training server system 140 deploys the trained model(s) during runtime.

By iteratively processing each data set corresponding to each historical patient, the model may be iteratively refined to generate accurate calibration-related determinations for a patient.

### Additional Considerations

The methods disclosed herein comprise one or more steps or actions for achieving the methods. The method steps and/or actions may be interchanged with one another without departing from the scope of the claims. In other words, unless a specific order of steps or actions is specified, the order and/or use of specific steps and/or actions may be modified without departing from the scope of the claims.

As used herein, a phrase referring to "at least one of" a list of items refers to any combination of those items, including single members. As an example, "at least one of: a, b, or c" is intended to cover a, b, c, a-b, a-c, b-c, and a-b-c, as well as any combination with multiples of the same element (e.g., a-a, a-a-a, a-a-b, a-a-c, a-b-b, a-c-c, b-b, b-b-b, b-b-c, c-c, and c-c-c or any other ordering of a, b, and c).

The previous description is provided to enable any person skilled in the art to practice the various aspects described herein. Various modifications to these aspects will be readily apparent to those skilled in the art, and the generic principles defined herein may be applied to other aspects. Thus, the claims are not intended to be limited to the aspects shown herein, but is to be accorded the full scope consistent with the language of the claims, wherein reference to an element in the singular is not intended to mean "one and only one" unless specifically so stated, but rather "one or more." Unless specifically stated otherwise, the term "some" refers to one or more. All structural and functional equivalents to the elements of the various aspects described throughout this disclosure that are known or later come to be known to those of ordinary skill in the art are expressly incorporated herein by reference and are intended to be encompassed by the claims. Moreover, nothing disclosed herein is intended to be dedicated to the public regardless of whether such disclosure is explicitly recited in the claims. No claim element is to be construed under the provisions of 35 U.S.C. §112(f) unless the element is expressly recited using the phrase "means for" or, in the case of a method claim, the element is recited using the phrase "step for."

While various examples of the invention have been described above, it should be understood that they have been presented by way of example only, and not by way of limitation. Likewise, the various diagrams may depict an example architectural or other configuration for the disclosure, which is done to aid in understanding the features and functionality that can be included in the disclosure. The disclosure is not restricted to the illustrated example architectures or configurations, but can be implemented using a variety of alternative architectures and configurations. Additionally, although the disclosure is described above in terms of various example examples and aspects, it should be understood that the various features and functionality described in one or more of the individual examples are not limited in their applicability to the particular example with which they are described. They instead can be applied, alone or in some combination, to one or more of the other examples of the disclosure, whether or not such examples are described, and whether or not such features are presented as being a part of a described example. Thus the breadth and scope of the present disclosure should not be limited by any of the above-described example examples.

All references cited herein are incorporated herein by reference in their entirety. To the extent publications and patents or patent applications incorporated by reference contradict the disclosure contained in the specification, the specification is intended to supersede and/or take precedence over any such contradictory material.

Unless otherwise defined, all terms (including technical and scientific terms) are to be given their ordinary and customary meaning to a person of ordinary skill in the art, and are not to be limited to a special or customized meaning unless expressly so defined herein.

Terms and phrases used in this application, and variations thereof, especially in the appended claims, unless otherwise expressly stated, should be construed as open ended as opposed to limiting. As examples of the foregoing, the term 'including' should be read to mean 'including, without limitation,' 'including but not limited to,' or the like; the term 'comprising' as used herein is synonymous with 'including,' 'containing,' or 'characterized by,' and is inclusive or open-ended and does not exclude additional, unrecited elements or method steps; the term 'having' should be interpreted as 'having at least;' the term 'includes' should be interpreted as 'includes but is not limited to;' the term 'example' is used to provide example instances of the item in discussion, not an exhaustive or limiting list thereof; adjectives such as 'known', 'normal', 'standard', and terms of similar meaning should not be construed as limiting the item described to a given time period or to an item available as of a given time, but instead should be read to encompass known, normal, or standard technologies that may be available or known now or at any time in the future; and use of terms like 'preferably,' 'preferred,' 'desired,' or 'desirable,' and words of similar meaning should not be understood as implying that certain features are critical, essential, or even important to the structure or function of the invention, but instead as merely intended to highlight alternative or additional features that may or may not be utilized in a particular example of the invention. Likewise, a group of items linked with the conjunction 'and' should not be read as requiring that each and every one of those items be present in the grouping, but rather should be read as 'and/or' unless expressly stated otherwise. Similarly, a group of items linked with the conjunction 'or' should not be read as requiring mutual exclusivity among that group, but rather should be read as 'and/or' unless expressly stated otherwise.

The term "comprising as used herein is synonymous with "including," "containing," or "characterized by" and is inclusive or open-ended and does not exclude additional, unrecited elements or method steps.

All numbers expressing quantities of ingredients, reaction conditions, and so forth used in the specification are to be understood as being modified in all instances by the term 'about.' Accordingly, unless indicated to the contrary, the numerical parameters set forth herein are approximations that may vary depending upon the desired properties sought to be obtained. At the very least, and not as an attempt to limit the application of the doctrine of equivalents to the scope of any claims in any application claiming priority to the present application, each numerical parameter should be construed in light of the number of significant digits and ordinary rounding approaches.

Furthermore, although the foregoing has been described in some detail by way of illustrations and examples for purposes of clarity and understanding, it is apparent to those skilled in the art that certain changes and modifications may be practiced. Therefore, the description and examples should not be construed as limiting the scope of the invention to the specific examples and examples described herein, but rather to also cover all modification and alternatives coming with the true scope and spirit of the invention.
The description can be further characterized by the following exemplary clauses:
1. An apparatus comprising:
   an analyte sensor;
   a memory comprising executable instructions; and
   a processor communicatively coupled to the memory, the processor configured to execute the instructions to cause the apparatus to:
      monitor, using the analyte sensor, an analyte of a patient during a time period to obtain measured analyte data for the analyte;
      monitor other measured sensor data indicative of a physiological state of the patient during the time period;
      determine, based on the physiological state of the patient during the time period, expected analyte data for the analyte;
      determine a correction factor based on the expected analyte data and the measured analyte data, the correction factor indicative of an error in calibration of the analyte sensor;
      determine whether recalibration of the analyte sensor is possible;
      if recalibration is possible, recalibrate the analyte sensor based on the correction factor; and
      if recalibration is not possible, recommend, to the patient, to replace the analyte sensor.
2. The apparatus of Clause 1, wherein the analyte is glucose.
3. The apparatus of Clause 1, wherein the physiological state comprises an oxygen level of the patient or a temperature of the patient.
4. The apparatus of Clause 1, wherein the expected analyte data is determined based on a mapping between historical analyte data for the analyte and the physiological state.
5. The apparatus of Clause 1, wherein the processor is further configured to determine that the measured analyte data deviates from the expected analyte data by an amount exceeding a threshold.
6. The apparatus of Clause 5, wherein determining the correction factor is in response to determining that the measured analyte data deviates from the expected analyte data by the amount exceeding the threshold.
7. The apparatus of Clause 5, wherein determining that the measured analyte data deviates from the expected analyte data by the amount exceeding the threshold comprises matching a datapoint from the analyte data with a datapoint from the expected analyte data based on a time when the datapoint from the analyte data was measured.
8. The apparatus of Clause 1, wherein the correction factor is based on an amount by which the measured analyte data deviates from the expected analyte data.
9. The apparatus of Clause 1, wherein the correction factor comprises an adjustment to a sensitivity of the analyte sensor or a rate of change of the sensitivity of the analyte sensor.
10. An apparatus comprising:
   a first analyte sensor;
   a second analyte sensor;
   a memory; and
   a processor communicatively coupled to the memory, the processor configured to:
      receive data indicative of a historical relationship between a first analyte and a second analyte of a patient during a first time period;
      monitor, using the first analyte sensor, the first analyte of the patient during a second time period to obtain first analyte data;
      monitor, using the second analyte sensor, the second analyte of the patient during the second time period to obtain second analyte data;
      determine, based on the first analyte data and the second analyte data, a current relationship between the first analyte and the second analyte; and
      determine that there is a substantial mismatch between the historical relationship and the current relationship, the substantial mismatch indicative of an error in calibration of the first analyte sensor or the second analyte sensor; and
      recalibrate the first analyte sensor or the second analyte sensor in response to determining that there is the substantial mismatch.
11. The apparatus of Clause 10, wherein the processor is further configured to determine that recalibration of the first analyte sensor or second analyte sensor is possible, wherein recalibrating the first analyte sensor or the second analyte sensor is in response to determining that recalibration of the first analyte sensor or second analyte sensor is possible.
12. The apparatus of Clause 10, wherein the first analyte is glucose and the second analyte is lactate.
13. An apparatus comprising:
   a first analyte sensor;
   a memory; and
   a processor communicatively coupled to the memory, the processor configured to:
      monitor, using the first analyte sensor, a first analyte of a patient during a time period to obtain first analyte data;
      determine a period of stability in the first analyte data;
      determine a calibration time based on the period of stability; and
      recalibrate the analyte sensor during the determined calibration time.
14. The apparatus of Clause 13, wherein the processor is further configured to provide a recommendation to recalibrate during the calibration time.
15. The apparatus of Clause 13, wherein during the period of stability, the first analyte data remains within a predefined range.
16. The apparatus of Clause 13, further comprising a second analyte sensor, wherein the processor is further configured to monitor, using the second analyte sensor, a second analyte of the patient during the time period to obtain second analyte data, and wherein the period of stability is further based on the second analyte data.
17. The apparatus of Clause 13, wherein the processor is further configured to receive measured physiological data indicative of a physiological state of the patient during the time period, wherein the period of stability is further based on the measured physiological data.
18. The apparatus of Clause 13, wherein the period of stability has a duration of at least five minutes.
19. The apparatus of Clause 13, wherein the calibration time occurs during the period of stability.
20. The apparatus of Clause 13, wherein determining the calibration time is further based on a time of insertion of the first analyte sensor into the patient.

## Claims

1. An apparatus comprising:
a first analyte sensor;
a second analyte sensor;
a memory; and
a processor communicatively coupled to the memory, the processor configured to:
receive data indicative of a historical relationship between a first analyte and a second analyte of a patient during a first time period;
obtain first analyte data during a second time period from the first analyte sensor;
obtain second analyte data from the second analyte sensor during the second time period;
determine a current relationship between the first analyte and the second analyte based on the first analyte data and the second analyte data;
determine a difference between the historical relationship and the current relationship; and
calibrate the first analyte sensor or the second analyte sensor based on the determined difference.

2. The apparatus of claim 1, wherein the processor is further configured to determine that recalibration of the first analyte sensor or second analyte sensor is possible, wherein recalibrating the first analyte sensor or the second analyte sensor is in response to determining that recalibration of the first analyte sensor or second analyte sensor is possible.

3. The apparatus of claim 1, wherein the first analyte is glucose and the second analyte is lactate.

4. The apparatus of claim 1 or claim 2, wherein the first analyte and/or the second analyte include glucose, lactate, ketones, glycerol, or electrolytes such as sodium and potassium.

5. The apparatus of claim 1 or claim 2, wherein the first analyte comprises glucose, and the second analyte comprises one or more of lactate, ketones, glycerol, electrolytes such as sodium and potassium.

6. The apparatus of any preceding claim, wherein the processor is further configured to monitor other measured sensor data indicative of a physiological state of the patient during the first or second time period.

7. The apparatus of clause 6, wherein the physiological state comprises an oxygen level of the patient or a temperature of the patient.

8. The apparatus of claim 6, wherein the other measured sensor data includes one or more of accelerometer data, gyrometer data, acoustic data, global positioning system (GPS) data, heart rate, heart rate reserve, heart rate variability (HRV), electrocardiogram (EKG) data, sweat, electromyogram (EMG), respiration rate, temperature, blood pressure, galvanic skin response, oxygen uptake data (e.g., V0₂ max), sleep, and impedance data.

9. The apparatus of claim 8, wherein the other measured sensor data is utilized to calculate metrics.

10. The apparatus of claim 9, wherein metrics include glucose levels, glucose thresholds, glucose trends, lactate levels, lactate baseline, lactate threshold, lactate trends, ketone levels, ketone production rates, insulin sensitivity, insulin on board, meal state, meal habits, physical fitness, metabolic rate, body temperature, blood pressure, heart rate, heart rate variability, respiratory rate, or blood-alcohol concentration.

11. The apparatus of any of claims 6 to 10, wherein the processor is further configured to determine whether a compression event has occurred based on the first and second analyte data and the physiological state of the patient.

12. The apparatus of claim 11, wherein the processor is further configured to provide an alert or notification to the user that a compression event is detected.

13. The apparatus of any preceding claim, wherein the processor is further configured to determine a wear location of the first sensor and/or second sensor based on at least the measured first analyte data.

14. The apparatus of any preceding claim, wherein the processor is further configured to determine a wear location of the first sensor and/or second sensor based on secondary sensor data.

15. The apparatus of any preceding claim, wherein the processor is further configured to determine a wear location of the first sensor and/or second sensor based on accelerometer data.
